# EUROPÄISCHE PATENTSCHRIFT

(11) **EP 1 555 290 B1**
(45) Veröffentlichungstag und Bekanntmachung des Hinweises auf die Patenterteilung: **27.06.2007**
(21) Anmeldenummer: 05000817.6
(22) Anmeldetag: 17.01.2005
(51) Int. Cl.: C08K 9/12, C08K 9/04, A61K 6/083, C08G 61/08

(54) **Dentalmaterialien auf der Basis von RÖMP-Kompositen**
Dental materials based on ROMP-composites
Matériel dentaire sur la base de composites ROMP

(30) Priorität: 15.01.2004 DE 102004002178
(43) Veröffentlichungstag der Anmeldung: 20.07.2005
(73) Patentinhaber: Ivoclar Vivadent AG, 9494 Schaan (LI)
(72) Erfinder: Smolak, Sonja, Dr., 80805 München (DE); Moszner, Norbert, Prof. Dr., FL - 9495 Triesen (LI); Stelzer, Franz, Prof. Dr., 8020 Graz (AT); Rheinberger, Volker M., Dr., 9490 Vaduz (LI)
(74) Vertreter: UEXKÜLL & STOLBERG

(56) Entgegenhaltungen:
- EP-A- 1 025 830
- JORDI M.A., SEERY T.A.P.: "The preparation of silica-poly(norbornene) nanocomposites by surface initiated polymerization" POLYMER PREPRINTS, Bd. 44, Nr. 1, 2003, Seiten 542-543, XP009046505
- BARRETT A.G.M, CRAMP S.M., ROBERTS R.S.: "ROMP-spheres: a novel high-loading polymer support using cross metathesis between vinyl polystyrene and norbornene derivatives" ORGANIC LETTERS, Bd. 1, Nr. 7, 1999, Seiten 1083-1086, XP002324915
- BUCHMEISER M.R., SINNER F., MUPA M., WURST K.: "Ring-opening metathesis polymerization for the preparation of surface-grafted polymer supports" MACROMOLECULES, Bd. 33, 2000, Seiten 32-39, XP002324916
- NAMYONG Y KIM ET AL: "Surface-Initiated Ring-Opening Metathesis Polymerization on Si/SiO2" MACROMOLECULES, AMERICAN CHEMICAL SOCIETY. EASTON, US, Bd. 33, Nr. 8, 18. April 2000 (2000-04-18), Seiten 2793-2795, XP002169336 ISSN: 0024-9297

## Beschreibung

Die vorliegende Erfindung betrifft füllstoffhaltige Zusammensetzungen auf der Basis von Monomeren, die durch ringöffnende Metathese polymerisiert werden können. Die Zusammensetzungen enthalten einen Initiator für die ringöffnende Metathesepolymerisation der chemisch oder physikalisch an den Füllstoff gebunden ist, und eignen sich besonders als Dentalmaterialien.

Die Polymerisation von Monomeren und Monomermischungen ist meist mit einer mehr oder weniger großen Volumenkontraktion verbunden. Dieser Polymerisationsschrumpf wirkt sich nachteilig auf die Dimensionsstabilität und die mechanischen Eigenschaften von Formkörpern aus. Bei Adhäsiven und Klebeverbindungen beeinträchtigt er die Haftungseigenschaften und Verbundfestigkeit, was bei Dentalwerkstoffen die Ausbildung von Spalten zwischen Zahn und Füllung und damit die Bildung von Sekundärkaries begünstigt.

Die Ringöffnungspolymerisation ist in der Regel mit einem geringen Polymerisationsschrumpf verbunden, und es wurde daher des öfteren versucht, diese zur Herstellung von Dentalmaterialien nutzbar zu machen.

Die US 4 387 215 offenbart Spiroorthoester, Spiroorthocarbonate und polycyclische Ketallactone, welche sich ringöffnend polymerisieren lassen und welche bei der Polymerisation keinen Schrumpf und teilweise sogar eine Expansion zeigen sollen.

Die WO 94/00501 beschreibt ein Verfahren zur Herstellung von Polymeren, welche sich wiederholende Spiroorthoestergruppen enthalten und sich zur Herstellung von Dentalmaterialien eignen sollen.

Die DE 195 06 222 A1 offenbart kationisch polymerisierbare, schrumpfungsarme Materialien für medizinische Anwendungen und dentale Zwecke auf der Basis von Oxetan- und Oxacyclobutan-Derivaten.

Die DE 44 39 485 C2 betrifft bicycloaliphatische 2-Methylen-1,3-dioxepane, die sich radikalisch und kationisch polymerisieren lassen.

In der US 5, 665 839 werden radikalisch polymerisierbare Oxathiepane beschrieben, die die Herstellung von Polymeren mit Ester, Amid oder Thioestergruppen erlauben.

Die DE 196 12 004 A1 offenbart multifunktionelle Vinylcyclopropan-Derivate, die sich besonders zur Herstellung von Dentalmaterialien eignen. Die Vinylcyclopropane sind radikalisch polymerisierbar und bilden Polymere ohne hydrolytisch spaltbare Gruppen in der Hauptkette.

Den obigen Monomeren ist gemeinsam, daß ihre Synthese aufwendig und teuer ist. Darüber hinaus sind Monomere wie Spiroorthocarbonate, Spiroorthoester oder 2-Methylen-1,3-dioxepane feuchtigkeitsempfindlich und weisen bei Raumtemperatur und in Gegenwart von SiO₂ oder silikatischen Füllstoffen nur eine begrenzte Stabilität auf.

Die DE 196 08 316 A1 betrifft durch (Meth)acrylatgruppen substituierte Norbornenylderivate, die durch ringöffnende Metathesepolymerisation (RÖMP) polymerisiert werden können und sich besonders zur Herstellung von Dentalmaterialien eignen.

Aus der EP 0 796 607 A2 sind funktionalisierte Polymere bekannt, die durch ringöffnende Metathesepolymerisation von Norbornenderivaten zugänglich sind. Diese Polymere weisen Carbonsäuregruppen und polymerisierbare Gruppen auf und.lassen sich durch radikalische Polymerisation vernetzen. Die Polymere eignen sich zur Herstellung von Adhäsiven und Beschichtungsmaterialien und können mit ionenfreisetzenden Füllstoffen Kompositzemente bilden.

Die EP 0 904 766 A2 offenbart durch RÖMP härtbare Dentalmassen auf der Basis von Monomeren mit ungesättigten cyclischen oder polycyclischen Resten, Füllstoff und einem Initiator für die ringöffnende Metathesepolymerisation. Die Materialien sollen sich durch einen raschen Polymerisationsverlauf und einen geringen Volumenschrumpf auszeichnen.

Die EP 0 904 767 A2 betrifft Dentalwerkstoffe auf der Basis von Oligomeren und Polymeren, die ihrerseits durch ringöffnende Metathesepolymerisation erhältlich sind.

Die DE 199 05 093 A1 offenbart lagerstabile und feuchtigkeitsunempfindliche Dentalmaterialien auf der Basis von bicyclischen Ringsystemen wie z.B. Bicyclo[2.2.1]heptenderivaten, die durch RÖMP polymerisierbar sind.

In der US 6,455,029 werden Abformmaterialien beschrieben, die durch RÖMP härtbare, mit Norbornenylgruppen funktionalisierte telechele Oligomere oder Polymere auf Basis von Polydimethylsiloxan enthalten.

Die WO 00/61288 offenbart funktionalisierte Trägermaterialien für chromatographische Anwendungen, die durch Derivatisierung organischer oder anorganischer Träger mit polymerisierbaren Gruppen zugänglich sind. Die polymerisierbaren Gruppen werden mit einem der Metathesepolymerisation zugänglichen Monomer im Zuge einer Pfropfpolymerisation umgesetzt. Das Monomer weist Gruppierungen oder Substituenten auf, die für die chromatographischen Trenneigenschaften verantwortlich sind.

Aus der WO 02/14376 sind immobilisierte Metathesekatalysatoren bekannt, die sich leicht vom Reaktionsmedium abtrennen lassen sollen. Ein typisches Anwendungsgebiet für diese Katalysatoren sind ringbildende Metathesereaktionen.

Schürer et al., Angew. Chem. 2000, 112, 4062, offenbaren einen polymergebundene Metatheseinitiator für die Ringschlußmetathese, der von der Reaktionslösung leicht abtrennbar ist und auch wiederverwendet werden kann. Im Vergleich zu ungebundenen Katalysatoren wurde eine niedrigere Aktivität gefunden, wofür Diffusionsprobleme zum aktiven Zentrum verantwortlich sind. Polymerisationen mit diesem Initiator wurden keine beschrie-ben.

In einem Übersichtsartikel widmet sich A. Fürstner, Angew. Chem. 2000, 112, 3140-3172, der Olefinmetathese. Er bestätigt die geringere Aktivität immobilisierter Katalysatoren.

Ein grundlegendes Problem der ringöffnenden Metathesepolymerisation ist darin zu sehen, daß die verwendeten Initiatoren nur schlecht in flüssigen, RÖMP-polymerisierbaren Monomeren löslich sind, was eine homogene Verteilung der Initiatoren erschwert und damit einerseits Inhomogenitäten zur Folge hat und andererseits höhere Initiatormengen erforderlich macht. Da Metatheseinitiatoren meist intensiv gefärbte Verbindungen sind, werden gefärbte Materialien enthalten, die für dentale Anwendungen insbesondere in sichtbaren Bereichen ungeeignet sind.

Darüber hinaus ist die Beständigkeit herkömmlicher, durch RÖMP härtbarer Materialien gegenüber Feuchtigkeit nicht zufriedenstellend, da häufig eine Verschlechterung der mechanischen Eigenschaften durch Wassereinlagerung beobachtet wird.

Der Erfindung liegt die Aufgabe zugrunde, füllstoffhaltige Materialien zur Verfügung zu stellen, die sich durch ringöffnende Metathesepolymerisation härten lassen ohne die obigen Nachteile aufzuweisen und die sich insbesondere zur Verwendung als Dentalwerkstoffe eignen.

Diese Aufgabe wird erfindungsgemäß durch Zusammensetzungen gelöst, die
(a) mindestens ein Monomer und/oder Oligomer, das der ringöffnenden Metathesepolymerisation zugänglich ist,
(b) mindestens einen Füllstoff, und
(c) mindestens einen Initiator für die ringöffnende Metathesepolymerisation enthalten.

Die Zusammensetzungen sind dadurch gekennzeichnet, daß der Initiator in chemischer oder physikalischer Form an den Füllstoff gebunden ist.

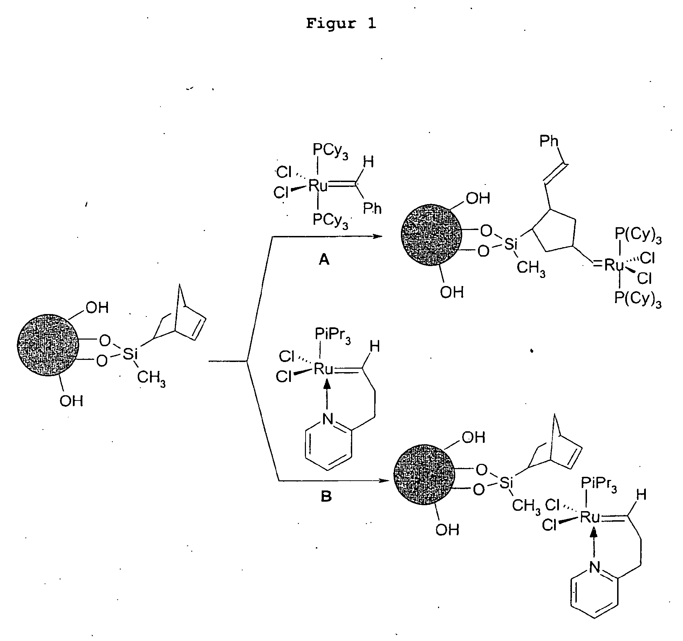

Figur 1 zeigt schematisch die Anbindung eines RÖMP-Initiators an einen Träger. Variante A zeigt einen kovalent gebundenen Initiator.

Variante B zeigt einen adsorptiv gebundenen, thermisch aktivierbaren Initiator. Die Füllstoffoberfläche ist mit einem Silan modifiziert, das eine metathesefähige Gruppe trägt. Diese reagiert bei der thermischen Aktivierung mit dem Initiator, wobei dieser kovalent an die Füllstoffoberfläche gebunden wird.

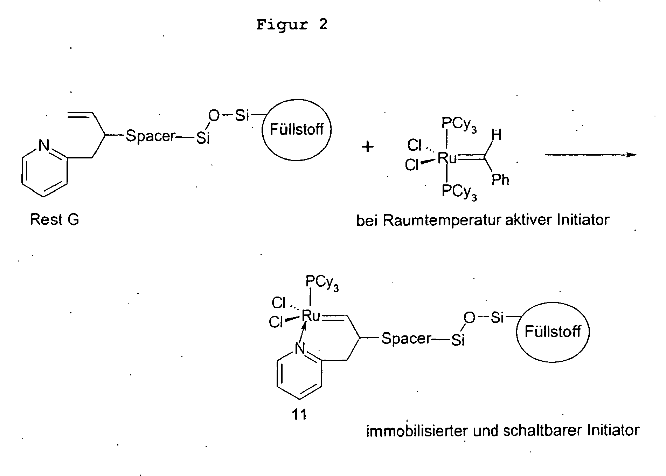

In Figur 2 ist schematisch ein aktivierbarer, kovalent gebundener Initiator gezeigt.

Zur Herstellung erfindungsgemäßer Zusammensetzungen wird der Füllstoff vorzugsweise mit einem Bindemittel behandelt, das der Anbindung des Initiators an den Füllstoff dient. Anschließend wird der Füllstoff mit dem Initiator beladen, der durch kovalente oder physikalische Wechselwirkungen an den Füllstoff gebunden wird. Schließlich wird der mit Initiator beladene Füllstoff mit Monomer gemischt. Die beschriebene Reihenfolge der Verfahrensschritte ist nicht zwingend. Beispielsweise kann der Initiator auch zunächst mit einem geeigneten Bindemittel umgesetzt und dann an den Füllstoff gebunden werden. Zusammensetzungen, die Füllstoff und ein polymerisierbares oder polymeres Matrixmaterial enthalten, werden auch als Komposite bezeichnet.

Gemäß einer bevorzugten Ausführungsform wird der Initiator mittels eines Silans der Formel (1) an den Füllstoff gebunden:

[(G-X)ᵣ-R¹-Y-R²]ₓ-SiR³ _{y}R⁴ _{z} Formel 1

in der die Variablen die folgenden Bedeutungen haben:
- G =: ein metathesefähiger, linearer oder verzweigter organischer Rest mit m Kohlenstoffatomen, wobei m eine ganze Zahl von 1 bis 40 ist, und 0 bis (m-2) Heteroatomen, ausgewählt aus N, O, Si, P und S, oder
ein metathesefähiger, cyclischer oder polycyclischer, cycloaliphatischer oder aromatischer organischer Rest mit m' Kohlenstoffatomen, wobei m' eine ganze Zahl von 3 bis 63 ist, und 0 bis (m'-2) Heteroatomen, ausgewählt aus N, O, Si, P und S, ist.

Die oben definierten aliphatischen Gruppen G enthalten vorzugsweise maximal 15 Heteroatome, die cyclischen Gruppen G maximal 28 Heteroatome, vorausgesetzt, daß gemäß obiger Definition eine solche Anzahl an Heteroatomen möglich ist.

Die Kohlenstoff- und Heteroatome der Gruppe G können im Falle aliphatischer Gruppen 1 bis (m-2) oder im Falle cyclischer Gruppen 1 bis (m'-2) Carbonylgruppen bilden. Die maximale Anzahl an Carbonylgruppen beträgt bei aliphatischen Gruppen vorzugsweise maximal 10, bei cyclischen Gruppen vorzugsweise maximal 15, vorausgesetzt, daß gemäß obigen Definitionen eine entsprechende Anzahl an Kohlenstoff- und Heteroatomen vorhanden ist.
- X,Y=: unabhängig voneinander -(C(=O)-O-, -C(=O)-N-, -O-C(=O)-O-, -O-C(=O)-NR⁵-, -CR⁵=N-, -O-, -S-, mit R⁵ = H, C₁-C₆-Alkyl, Benzyl oder entfällt;
- R¹ =: ein (r+1)-wertiger, linearer oder verzweigter aliphatischer, cycloaliphatischer oder aromatischer organischer Rest C₂-C₁₀-Rest oder entfällt;
- R² =: eine C₁-C₅-Alkylengruppe oder entfällt;
- R³ =: Halogen, Hydroxy, eine C₁-C₅-Alkoxy- oder C₁-C₅-Acyloxygruppe;
- R⁴ =: C₁-C₁₂-Alkyl, C₃-C₁₂-Cyloalkyl oder Phenyl;
- r =: 1, 2 oder 3;
- x =: 1, 2 oder 3;
- y =: 1, 2 oder 3;
- z =: 0, 1 oder 2,
wobei die Summe der Variablen x+y+z gleich 4 ist.

Unter Halogen wir hierin, wenn nicht anders angegeben, vorzugsweise Chlor, Brom oder Iod verstanden.

Als metathesefähige Gruppen G sind ungesättigte Gruppen, die bevorzugt endständige C-C-Doppel- oder C-C-Dreifachbindungen aufweisen, geeignet, wie z.B. Vinyl-, Allyl- oder Alkinylgruppen, vorzugsweise ungesättigte cyclische Gruppen wie z.B. Cyclooctenyl- oder besonders bevorzugt Norbornenyl-Gruppen. Demgegenüber sind (Meth)acrylat-Gruppen nicht geeignet.

Besonders bevorzugt sind Silane der Formel (1), in der die Variablen die folgenden Bedeutungen haben:
- G =: ein Rest der Formel (2) 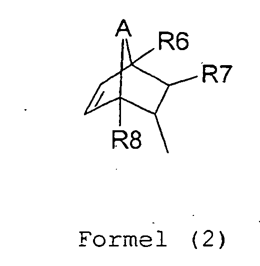 in der die Variablen wie folgt definiert sind:
A = O, NH, S, ein gesättigter oder ungesättigter organischer Rest mit t Kohlenstoffatomen, wobei t eine ganze Zahl von 1 bis 12 ist, der 0 bis (t-1) Heteroatome, ausgewählt aus N, O, Si, P und S, enthalten kann;
R⁶, R⁷, R⁸ = unabhängig von einander jeweils ein linearer oder verzweigter aliphatischer Rest mit q Kohlenstoffatomen, wobei q eine ganze Zahl von 1 bis 15 ist, ein cyclischer oder polycyclischer organischer Rest mit q' Kohlenstoffatomen, wobei q' eine ganze Zahl von 3 bis 15 ist, und 0 bis (q-1) bzw. 0 bis (q'-1) Heteroatomen, ausgewählt aus N, O Si, P, und S;
- X,Y =: entfallen;
- R¹ =: ein linearer C₁- bis C₅₋ Alkylenrest oder entfällt;
- R² =: entfällt;
- R³ =: Chlor, Methoxy, Ethoxy;
- R⁴ =: Methyl;
- r =: 1;
- x =: 1;
- y =: 1, 2 oder 3;
- z =: 0, 1 oder 2;
wobei die Summe von x+y+z gleich 4 ist.

A und unabhängig davon R⁶, R⁷, R⁸ enthalten vorzugsweise maximal 7 Heteroatome, vorausgesetzt, daß die obigen Definitionen eine solche Zahl von Heteroatomen zulassen.

Die Kohlenstoff- und Heteroatome von R⁶, R⁷, R⁸ können 1 bis (q-1) bzw. (q'-1) Carbonylgruppen bilden. Die maximale Anzahl an Carbonylgruppen beträgt vorzugsweise maximal 5, vorausgesetzt, daß die obigen Definitionen eine solche Anzahl an Kohlenstoff- und Heteroatomen zulassen.

Die bevorzugten Bedeutungen der einzelnen Variablen können unabhängig voneinander ausgewählt werden. Silane, bei denen 2 oder mehr, vorzugsweise alle Variablen eine der bevorzugten Bedeutungen aufweisen, sind naturgemäß ganz besonders bevorzugt.

Demgemäß sind besonders bevorzugte Verbindungen der Formel 1 Norborn-2-en-5-yl-trichlorsilan, Norborn-2-en-5-yl-triethoxysilan, Norborn-2-en-5-yl-dimethylchlorsilan, Norborn-2-en-5-yl-dimethylethoxysilan, Norborn-2-en-5-yl-methyldichlorsilan und Norborn-2-en-5-yl-methyldiethoxysilan.

Gemäß einer weiteren Ausführungsform erfolgt die Anbindung des Initiators an den Füllstoff auf physikalische Weise. Hierzu wird der Füllstoff vorzugsweise mit einem Silan behandelt, das keine metathesefähigen Gruppen aufweist. Vorzugsweise erfolgt die physikalische Anbindung mittels eines Silans der Formel (3), in der die Variablen die folgenden Bedeutungen haben:

(R⁹)ₐ(R¹⁰)_{b}(R¹¹)_{c}Si(R¹²)_{d} Formel (3)

- R¹² =: Halogen, Hydroxy, eine C₁-C₅-Alkoxy- oder C₁-C₅-Acyloxygruppe
- R⁹, R¹⁰, R¹¹ =: unabhängig voneinander ein gesättigter oder ungesättigter, linearer oder verzweigter organischer Rest mit s Kohlenstoffatomen, wobei s eine ganze Zahl von 2 bis 20 ist, und 0 bis (s-1) Heteroatomen, ausgewählt aus N, O, Si, P und S; oder
ein gesättigter, ungesättigter oder aromatischer, cyclischer oder polycyclischer organischer Rest mit s' Kohlenstoffatomen, wobei s' eine ganze Zahl von 3 bis 15 ist, mit 0 bis (s'-1) Heteroatomen, ausgewählt aus N, O, Si, P und S;
- a =: 0, 1, 2 oder 3;
- b =: 0, 1, 2 oder 3;
- c =: 0, 1, 2 oder 3;
- d =: 1, 2 oder 3

Die Summe aus a+b+c+d beträgt 4.

Die Reste R⁹, R¹⁰, R¹¹ enthalten vorzugsweise maximal 7 Heteroatome, vorausgesetzt daß die obigen Definitionen eine solche Anzahl an Heteroatomen zulassen.

Die Kohlenstoff- und Heteroatome von R⁹, R¹⁰, R¹¹ können 1 bis (s-1) bzw. (s'-1) Carbonylgruppen bilden. Die maximale Anzahl an Carbonylgruppen beträgt vorzugsweise maximal 5, vorausgesetzt, daß die obigen Definitionen eine solche Anzahl an Kohlenstoff- und Heteroatomen zulassen.

Bevorzugte Reste für R⁹, R¹⁰, R¹¹ sind, unabhängig voneinander, Methyl, Ethyl, Propyl, Cyclohexyl oder Phenyl.

Besonders bevorzugte Silane der Formel (3) sind Verbindungen, die neben Halogenresten oder Alkoxygruppen ausschließlich Alkylreste tragen, wie Trimethylchlorsilan oder Trimethylalkoxysilan.

Die Silane der Formel (1) ermöglichen eine physikalische oder chemische Anbindung des Initiators an die Füllstoffoberfläche. Die Silane reagieren einerseits unter Abspaltung von R³ mit Hydroxylgruppen der Füllstoffoberfläche und andererseits über die metathesefähigen Gruppen mit dem Initiator, so daß dieser kovalent an den Füllstoff gebunden wird. Initiatoren, die bei Raumtemperatur aktiv sind, reagieren bereits beim Beladen des Füllstoffs mit den metathesefähigen Gruppen. Photochemisch oder thermisch aktivierbare Initiatoren werden an der Füllstoffoberfläche adsorbiert. Die Reaktion mit den metathesefähigen Gruppen der Füllstoffoberfläche findet in diesem Fall erst nach der entsprechenden Aktivierung statt.

Als Füllstoffe sind die in der Dentaltechnik verwendeten, anorganischen partikulären Füllstoffe, z.B. Pulver von röntgenopaken Gläsern oder hochdisperse Kieselsäure, bevorzugt. Für die erfindungsgemässen Dentalmaterialien können als Füllstoffe vor allem anorganische partikuläre Füllstoffe, wie mikrofeine Füllstoffe mit einer Primärpartikelgröße von 5,0 bis 500 nm, z.B. pyrogene Kieselsäure oder Fällungskieselsäure oder Mischoxide aus SiO₂, ZrO₂, TaO₂, La₂O₃, Yb₂O₃ und/oder CeO₂, sowie Makro- oder Minifüllstoffe mit einer durchschnittlichen Teilchengröße von 0,01 bis 5 µm, wie Quarz-, Glaskeramik- oder Glaspulver, sowie röntgenopake Füllstoffe, wie Ytterbiumtrifluorid, eingesetzt werden.

Die Oberflächenmodifizierung der Füllstoffe wird im Fall der oben beschriebenen Silane in Suspension in einem trockenen organischen Lösungsmittel, z.B. Toluol oder Xylol durchgeführt. Die hydrolytischen Spaltprodukte werden aus dem Reaktionsgemisch entfernt, indem z.B. bei der Verwendung von Chlorsilanen tertiäre Amine als Säurefänger zugesetzt werden. Bei der Verwendung von Alkoxysilanen wird der entstehende Alkohol aus dem Reaktionsgemisch destillativ abgetrennt. Zur Aufarbeitung wird der modifizierte Füllstoff abzentrifugiert, gewaschen und im Väkuum getrocknet.

Die Anbindung der Silane an den Füllstoff erfolgt, nach Hydrolyse der labilen Silizium-Chlor bzw. Silizium-Alkoxy-Bindung durch oberflächlich adsorbiertes Wasser zu Silanolen, in einer Kondensationsreaktion mit den Silanol-Gruppen des Füllstoffs (P. van der Voort, E. F. Vansant, J. Liqu. Chrom. Rel. Technol. 1996, 19, 2723).

Die Menge an Silanisierungsmittel wird vorzugsweise so gewählt, daß der Gehalt an metathesefähigen Gruppen am Füllstoff 0,0001-1,0 mmol pro Gramm Füllstoff und besonders bevorzugt 0,01-0,3 mmol pro Gramm Füllstoff beträgt. Die maximale Menge an Silanisierungsmittel pro Gramm Füllstoff wird dabei durch die spezifischen Oberfläche des Füllstoffs und dessen SiO₂-Gehalt bestimmt. Je größer die spezifische Oberfläche oder der SiO₂-Gehalt ist, desto mehr Si-OH Gruppen sind vorhanden und desto mehr Silanisierungsmittel kann gebunden werden.

Bei reinem SiO₂ beträgt die spezifische Oberfläche vorzugsweise 20 und 200 m²/g, wobei SiO₂ mit einer spezifische Oberfläche von 40 bis 100 m²/g besonders bevorzugt ist. Im Falle von SiO₂-haltigen Gläsern beträgt der SiO₂-Gehalt vorzugsweise 10 und 95 Gew.-%, wobei ein SiO₂-Gehalt zwischen 30 und 60 Gew.-% besonders bevorzugt ist.

Der Umfang der Silanisierung läßt sich mittels C,H-Elementaranalyse bestimmen.

Der oberflächenmodifizierte Füllstoff wird mit einem Metatheseinitiator beladen. Der Metatheseinitiator kann dabei ein bereits bei Raumtemperatur aktiver Initiator sein, z.B. ein bei Raumtemperatur aktives Übergangsmetallcarben. In diesem Fall reagiert der Initiator bereits beim Mischen mit den zur Metathesereaktion fähigen Gruppen der Füllstoffoberfläche und wird kovalent an den Füllstoff gebunden, wie in Figur 1, Variante A gezeigt ist. Hierbei wird unterstellt, daß das Mischen der Komponenten gewöhnlich bei Raumtemperatur erfolgt.

Die Polymerisation startet bei Zugabe des Monomers von der Füllstoffoberfläche weg, der Initiator bleibt immer am Ende der wachsenden Polymerkette. Im Fall von thermisch oder photochemisch aktivierbaren Initiatoren setzt die Polymerisation erst nach der Aktivierung ein.

Bevorzugte bei Raumtemperatur aktive Initiatoren sind solche gemäß der allgemeinen Formel 4, in der die Variablen die folgenden Bedeutungen haben: 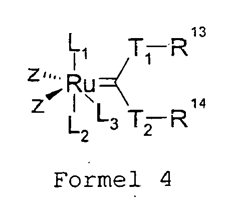
- Z =: Cl, Br, F, I, Tosylat oder eine der für L₁, L₂, L₃ angegebenen Bedeutungen;
- L₁, L₂, L₃ =: unabhängig voneinander P(R¹⁵)₃ mit R¹⁵ = Phenyl, Isopropyl, Cyclohexyl oder 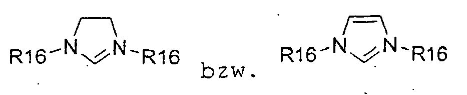 mit R¹⁶ = Mesityl (2,4,6-Trimethylphenyl; MES);
oder Pyridin, das unsubstituiert oder in Position 2 bzw in Position 2 und 4 mit Br, Cl, F, I, OCH₃ substituiert ist; oder entfällt;
- R¹³, R¹⁴ =: voneinander unabhängig H oder ein aromatischer oder aliphatischer, polycyclischer oder kondensierter C₆-C₂₀-Ring bzw. Ringsystem, mit 0-5 Heteroatomen im Ring, ausgewählt aus N, S, O, P, wobei der Ring bzw. das Ringsystem unsubstituiert ist oder zusätzlich mit 0-5 Substituenten des Typs -Cl, -Br, -I, -F, -OR¹⁷, -CH=N-R¹⁷, -C(=O)R¹⁷, -C(=O)OR¹⁷, -OC(=O)R¹⁷ substituiert sein kann, wobei R¹⁷ ein lineares oder verzweigtes, acyclisches C₁-C₁₄-Alkyl oder cyclisches C₃-C₁₄-Alkyl oder ein aromatischer Rest mit 6 bis 14 Kohlenstoffatomen ist, wobei R¹⁷ 0-5 Heteroatomen, ausgewählt aus.N, 0, Si, S enthalten kann;
- T₁, T₂ =: unabhängig voneinander -O-, -S-, oder ein gesättigtes C₁ bis C₄-Alkylen oder ungesättigtes C₂ bis C₄-Alkylen, welches zusätzlich 0-2 Heteroatome der Gruppe N, O, S, Si beinhalten kann oder entfällt.

Besonders bevorzugte bei Raumtemperatur aktive Metatheseinitiatoren sind die folgenden Verbindungen: 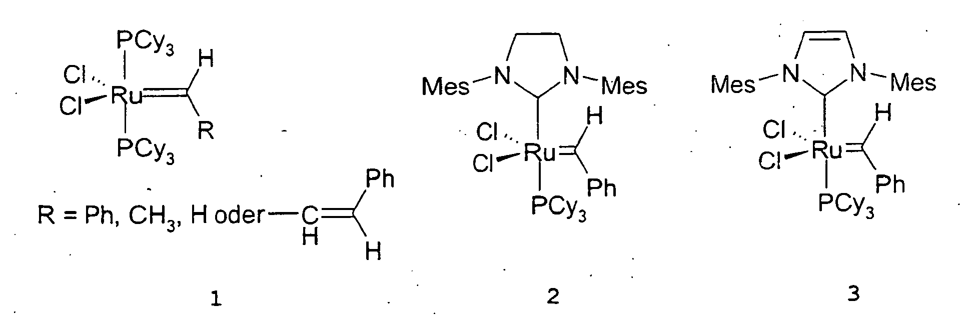 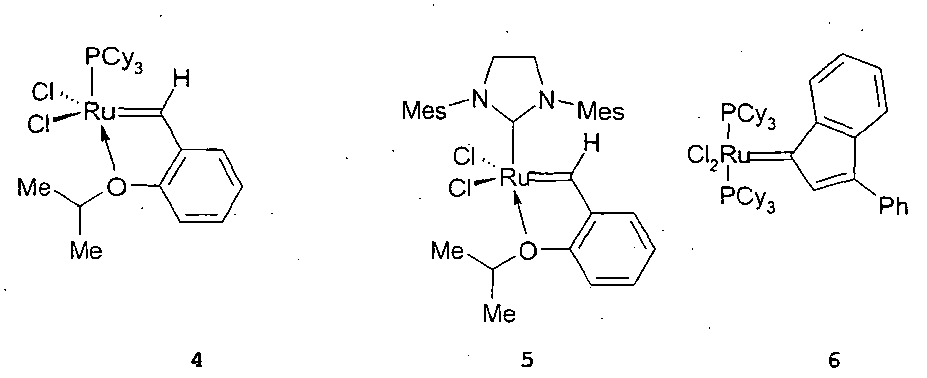 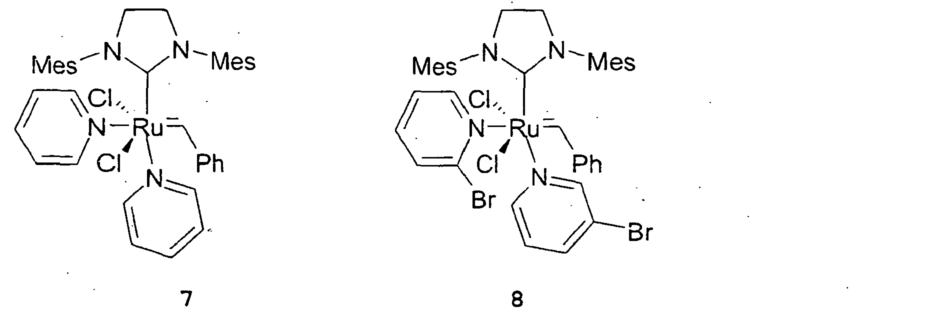 Cy = Cyclohexyl; Me = Methyl; MES = 2,4,6-Trimethylphenyl

Diese Initiatoren sich aus der Literatur bereits bekannt: **1:** T. S. Nguyen, L. K. Johnson, R. H. Grubbs, J. W. Ziller; J. Am. Chem. Soc. 1992, 114, 3974; P. Schwab, R. H. Grubbs, J. W. Ziller, J. Am. Chem .Soc. 1996, 118, 100; WO9706185. **2:** M. Scholl, S. Ding, C. W. Lee, R. H. Grubbs, Org. Lett. 1999, 953; WO0071554. **3:** M. Scholl, T. M. Trnka, J. P. Morgan, R. H. Grubbs, Tetrahedron. Lett. 1999, 40, 2247; T. Weskamp, F. J. Kohl, W. Hieringer, D. Gleich, W. A. Hermann, J. Organomet. Chem. 1999, 582, 362. **4**: J. S. Kingsbury, J. P. A. Harrity, P. J. Bonitatebus, A. H. Hoveyda, J. Am. Chem. Soc. 1999, 121, 791. **5**: S. B. Garber, J. S. Kingsbury, B. Gray, A. H. Hoveyda, J. Am. Chem. Soc. 2000, 122, 8168; WO0214376. **6:** L. Jafarpour, H.-J. Schanz, E. D. Stevens, S. P. Nolan, Organometallics 1999, 18, 5416; A. Fürstner, O. R. Thiel, L. Ackermann, H.-J. Schanz, S. P. Nolan, J. Org. Chem. 2000, 65, 2204. **7:** M. S. Sanford, J. A. Love, R. H. Grubbs, Organometallics, 2001, 20, 5314. **8:** J. A. Love, J. P. Morgan, T. M. Trnka, R. H. Grubbs, Angew. Chem., 2002, 114, 4207. Die Initiatoren **1, 2, 4** und **5** sind kommerziell bei Materia Inc., Pasadena erhältlich.

Vorzugsweise enthalten die erfindungsgemäßen Zusammensetzungen einen Initiator für die RÖMP, der thermisch oder photochemisch aktivierbar ist.

Bevorzugte thermisch und/oder photochemisch aktivierbare Initiatoren sind solche gemäß der allgemeinen Formel 5, in der die Variablen die folgenden Bedeutungen haben: 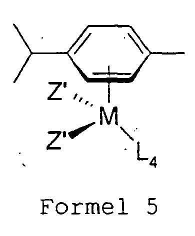
- M =: Os oder Ru;
- Z' =: Cl, Br, F, I, Tosylat oder eine der für L₄ angegebenen Bedeutungen;
- L₄ =: P (R¹⁸)₃ mit R¹⁸= Phenyl, Isopropyl, Cyclohexyl oder 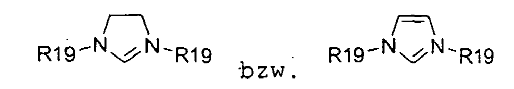 mit R¹⁹ = Mesityl (2,4,6-Trimethylphenyl).

Weitere bevorzugte thermisch bzw. photochemisch aktive Metatheseinitiatoren sind die folgenden Verbindungen:

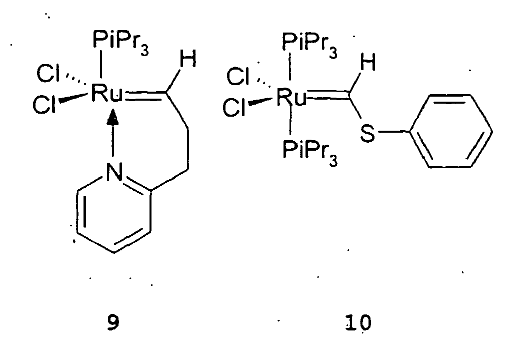

Die Initiatoren **9** und **10** sind thermisch und Initiatoren der Formel 5 photochemisch und thermisch initiierbar. Diese Initiatoren sich aus der Literatur bereits bekannt: **9** und **10:** P. A. van der Schaaf, R. Kolly, H-J. Kirner, F. Rime, A. Mühlebach, A. Hafner, J. Organometallic Chem., 2000, 606, 65; WO99/00397; Formel 5: A. Hafner, A. Mühlebach, P. A. van der Schaaf, Angew. Chem. 1997, 109, 2213; L. Delaude, A. Demonceau, A. F. Noels, Chem. Commun, 2001, 986.

Thermisch oder photochemisch schaltbare Initiatoren wie z.B. thermo- oder photolabile Verbindungen des Typs **9, 10** oder Formel 5 werden zunächst nur an der Füllstoffoberfläche adsorbiert, und nicht kovalent gebunden. Der mit metathesefähigen Gruppen modifizierte Füllstoff wird sozusagen mit dem Metatheseinitiator oberflächlich imprägniert, wie in Figur 1, Variante B gezeigt ist. Die Initiatormoleküle befinden sich allerdings in unmittelbarer Nähe der reaktiven, funktionellen Gruppen des Füllstoffs. Vermischt man diesen mit Initiator imprägnierten Füllstoff mit RÖMP-fähigem Monomer und aktiviert den schaltbaren Metatheseinitiator durch Zufuhr von Wärme bzw. Licht, so startet die Metathesepolymerisation. Durch die räumliche Nähe der adsorbierten Initiatormoleküle zu den metatheseaktiven Ankergruppen.des Füllstoffs werden diese in das Polymer eingebaut; es kommt schließlich auch ein kovalenter Verbund zwischen anorganischem Füllstoff und organischer Matrix zustande.

Es ist auch möglich, den Initiator über solche Silane nach Formel (1) an den Füllstoff zu binden, die neben einer oder mehreren metathesefähigen Gruppen G mindestens eine Gruppe aufweisen, die koordinative Bindungen zum Metallzentrum des Initiators ausbilden kann. Aus diese Weise lassen sich schaltbare, kovalent an den Füllstoff gebundene Initiatoren herstellen. Beispielsweise kann ein bei Raumtemperatur aktiver Initiator wie **1** oder **2,** wie in Figur 2 gezeigt, über eine Metathesereaktion an die metathesefähige Gruppe des silanisierten Füllstoffs angebunden werden. Gleichzeitig kann eine weiter vorhandene funktionelle Gruppe, z.B. eine Pyridinylgruppe einen Phosphinliganden verdrängen und an das Metallzentrum des Initiators koordinieren. Der so erhaltene Initiator **11** ist ein immobilisiertes Analogon zum schaltbaren Initiator **9.** Es ist auf diese Weise möglich, einen bei Raumtemperatur aktiven Initiator am Füllstoff zu immobilisieren und gleichzeitig in eine thermisch oder photochemisch schaltbare Form zu überführen.

Im Rahmen der vorliegenden Erfindung werden sowohl Initiatoren, die sofort kovalent an den Füllstoff gebunden werden, als auch Initiatoren, die erst nach entsprechender Aktivierung kovalent gebunden werden, als chemisch gebundene Initiatoren bezeichnet. Unter physikalisch gebundenen Initiatoren werden solche Substanzen verstanden, die auch nach der Härtung der Materialien nicht kovalent an den Füllstoff gebunden sind. Chemisch gebundene Initiatoren sind erfindungsgemäß bevorzugt.

Die Beladung des Füllstoffs mit dem Initiator erfolgt, indem man Füllstoff, vorzugsweise Füllstoff, der mit einem Bindemittel behandelt wurde, in einer Lösung des Metatheseinitiators in einem trockenen organischen Lösungsmittel bei Raumtemperatur suspendiert und rührt. Im Falle der bei Raumtemperatur aktiven Metatheseinitiatoren wird solange gerührt, bis der Initiator vollständig mit dem Reaktivfüller reagiert hat und die Lösung entfärbt ist. Das Lösungsmittel wird entfernt und der nun initiierende Füllstoff im Vakuum getrocknet und unter Inertgas gekühlt gelagert.

Im Falle der thermisch oder photochemisch aktivierbaren Initiatoren wird der Füller in einer Lösung des schaltbaren Metatheseinitiators bei Raumtemperatur, gegebenenfalls in Dunkelheit, suspendiert, gerührt und das Lösungsmittel unter vermindertem Druck abgezogen. Die mit Initiator beladenen Füllstoffe werden unter Inertgas gekühlt gelagert.

Als besonders geeignet zum Aufbringen des Initiators haben sich die Lösungsmittel Pentan, Cyclohexan, Heptan, Toluol, Xylol, Dichlormethan, Chloroform, Chlorbenzol, Aceton, THF und Diethylether erwiesen.

Die Beladung des Füllstoffs mit Initiator beträgt vorzugsweise zwischen 0,00001 und 0,1 mmol, vorzugsweise 0,0001 bis 0,05 mmol, und ganz besonders bevorzugt 0,001 bis 0,01 mmol Initiator pro Gramm Füllstoff.

Erfindungsgemäß werden vorzugsweise maximal 10 %, besonders bevorzugt 2 bis 10 % der metathesefähigen Gruppen am Füllstoff durch Initiator beladen.

Neben dem mit Initiator beladenen Füllstoff können die erfindungsgemäßen Zusammensetzungen zusätzlich einen Füllstoffanteil enthalten, der keinen Initiator trägt. Als weitere Füllstoffe eignen sich besonders Materialien an, die mit Gruppen modifiziert sind, die der ringöffnenden Polymerisation zugänglich sind, vorzugsweise einem Silan der Formel (1). Füllstoffe mit metathesefähigen Gruppen wirken bei der Polymerisation der Zusammensetzungen als Vernetzer und werden bei der RÖMP kovalent in das Polymernetzwerk eingebunden. Als Füllstoffmaterialien sind die oben definierten partikulären anorganischen Materialien bevorzugt.

Die Verwendung von initiatorfreien Füllstoffanteilen, die mit metathesefähigen Gruppen modifiziert sind, ist insbesondere dann bevorzugt, wenn der Initiator an Füllstoff gebunden ist, der mit einem Silan der Formel (3), d.h. einem Silan ohne metathesefähige Reste modifiziert ist. Zusammensetzungen, die ausschließlich Füllstoffe enthalten, die mit zur Metathesepolymerisation fähigen Gruppen modifiziert sind, sind jedoch besonders bevorzugt, da so alle Füllstoffanteile bei Metathesepolymerisation kovalent in den gehärteten Werkstoff eingebunden werden.

Der mit Initiator beladene Füllstoff wird mit mindestens einem Monomer und/oder Oligomer gemischt, die durch, RÖMP polymerisierbar sind.

Im Fall der bei Raumtemperatur aktiven Initiatoren startet die Polymerisation bereits beim Vermengen der Komponenten, so daß nur Zweikomponentensysteme realisierbar sind, in denen initiatorhaltiger Füllstoff und RÖMP Monomer getrennt auf zwei oder mehr unterschiedliche Komponenten verteilt sind. Kits (Bausätze) zur Herstellung erfindungsgemäßer Zusammensetzungen, die initiatorhaltigen Füllstoff und Monomer/Oligomer (a) in räumlich getrennter Form enthalten, sind ebenfalls Gegenstand der Erfindung. Erfindungsgemäße Kits können beispielsweise initiatorhaltigen Füller (Komponente 1) und eine dünnflüssige Paste aus oberflächenmodifiziertem Füller und Monomer/Oligomer (Komponente 2) oder initiatorhaltigen Füller (Komponente 1) und flüssiges Monomer/Oligomer oder Monomer/Oligomergemisch (Komponente 2) enthalten.

Im Falle thermisch oder photochemisch schaltbarer Initiatoren sind einkomponentige Systeme möglich, d.h. es kann eine bei Raumtemperatur lagerstabile Kompositpaste aus initiierendem Füllstoff, gegebenenfalls weiterem Füllstoff und Monomer/Oligomer hergestellt werden. Die Aushärtung der Mischung erfolgt erst durch Zufuhr von Wärme oder Bestrahlung mit Licht.

Als Komponente (a) werden vorzugsweise Monomere oder Monomermischungen verwendet. Als Monomere sind metathetisch polymerisierbare Ringsysteme, vorzugsweise bi- oder multicyclische Ringsysteme mit mindestens einer endocyclischen Doppelbindung bevorzugt. Besonders geeignet sind carbocyclische und heterocyclische Bicyclo[x',y',z']kohlenwasserstoffe, wobei x',y',z' unabhängig voneinander jeweils eine ganze Zahl von 1 bis 6 sind, bevorzugt x'=2, y'=2, z'=1.

Besonders bevorzugt eingesetzt werden Norbornenderivate, der allgemeinen Formeln (6) und/oder (7) 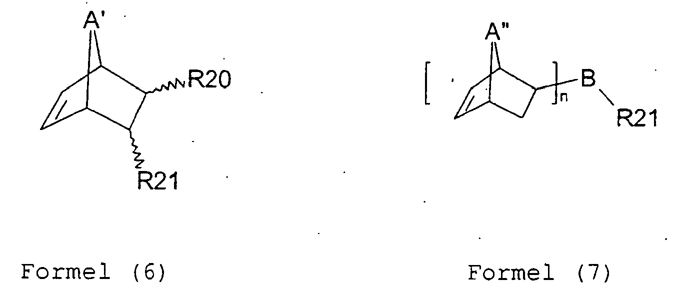 in denen die Variablen die folgenden Bedeutungen haben:
- A', A" =: O S, NH, ein gesättigter oder ungesättigter organischer Rest mit u Kohlenstoffatomen, wobei u eine ganze Zahl von 1 bis 15 ist, der 0 bis (u-1) Heteroatome, ausgewählt aus N, O, Si, P und S, enthalten kann;
- B =: -CH₂- oder entfällt;
- R²⁰, R²¹ =: H oder ein gesättigter oder ungesättigter organischer Rest mit v Kohlenstoffatomen, wobei v eine ganze Zahl von 1 bis 30 ist, und mit 0 bis (v-1) Heteroatomen aus der Gruppe N, O, Si, P, S und F; oder
R²⁰ und R²¹ bilden zusammen mit den Atomen, an die sie gebunden sind, ein ankondensiertes, gesättigtes oder ungesättigtes alicyclisches Ringsystem 4 bis 12 Kohlenstoffatomen oder ein ankondensiertes aromatisches Ringsystem mit 6 bis 12 Kohlenstoffatomen, das seinerseits durch C₁-C₆-Alkyl oder Benzyl substituiert sein kann;
- R²² =: n-fach substituiertes C₁- bis C₁₀-Alkylen, C₆- bis C₁₀-Arylen, -O-C(=O)-Phenylen-C(=O)-O-, (-C(=O)-)₄(C₆H₂), 2,4,6-Trioxo-1,3,5-triazinyl, -O-C(=O)-(CH₂)ₘ-C(=O)-O-; -O-C(=O)-NH-(CH₂)ₘ-NH-C(=O)-O-, wobei m eine ganze Zahl von 1 bis 10 ist;
- n =: eine ganze Zahl von 1 bis 4.

Im Fall der Gruppen A' und A'' beträgt die Anzahl der Heteroatome vorzugsweise maximal 10, vorausgesetzt, daß die obigen Definitionen eine solche Anzahl an Kohlenstoff- und Heteroatomen zulassen.

Die Kohlenstoff- und Heteroatome von A' und A'' können 1 bis (u-1) Carbonylgruppen bilden. Die maximale Anzahl an Carbonylgruppen beträgt vorzugsweise maximal 5, vorausgesetzt, daß die obigen Definitionen eine solche Anzahl an Kohlenstoff- und Heteroatomen zulassen.

Im Fall der Gruppen R²⁰ und R²² beträgt die Anzahl der Heteroatome vorzugsweise maximal 20, vorausgesetzt, daß die obigen Definitionen eine solche Anzahl an Kohlenstoff- und Heteroatomen zulassen.

Die Kohlenstoff- und Heteroatome von R²⁰, R²¹ können 1 bis (v-1) Carbonylgruppen bilden. Die maximale Anzahl an Carbonylgruppen beträgt vorzugsweise maximal 10, vorausgesetzt, daß die obigen Definitionen eine solche Anzahl an Kohlenstoff- und Heteroatomen zulassen.

Bevorzugte Definitionen für die einzelnen Variablen, die unabhängig voneinander ausgewählt werden können sind:
- A', A'' =: -CH₂-;
- B =: -CH₂- oder entfällt;
- R²⁰, R²¹ =: H, -C(=O)-OCH₃; -O-C(=O)-CH₃; -CH₂-O-C(=O)-CH₃; oder
R²⁰ und R²¹ bilden zusammen mit den Atomen, an die sie gebunden sind, ein ankondensiertes, gesättigtes oder ungesättigtes alicyclisches Ringsystem mit 5 bis 7 Kohlenstoffatomen oder einen sauerstoffhaltigen Heterocyclus mit 4 Kohlenstoffatomen und einem Sauerstoffatom, der unsubstituiert oder durch =O substituiert sein kann, wie beispielsweise einen ankondensierten Lactonring;
- R²²=: 1 bis 3-fach substituiertes C₁- bis C₆-Alkylen, ins- besondere C₁- bis C₃-Alkylen; -O-C(=O)-Phenylen-C (=O)-O-, -O-C(=O)-(CH₂)ₘ-C(=O)-O-; -O-C(=O)-NH-(CH₂)ₘ-NH-C(=O)-O-; wobei m eine ganze Zahl von 1 bis 6 ist;
- n =: eine ganz= Zahl von 1 bis 3.

Ganz besonders bevorzugte Monomere sind:

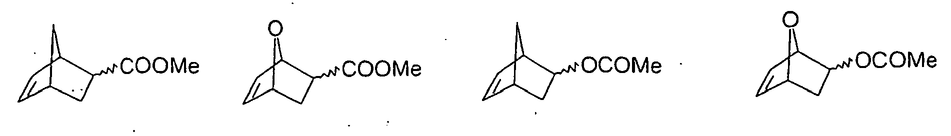

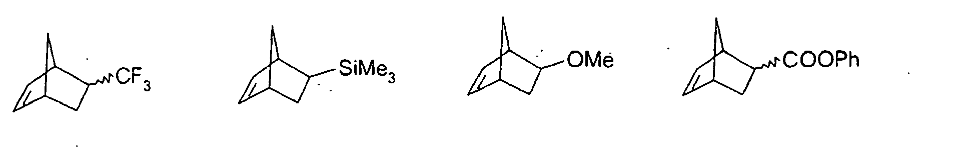

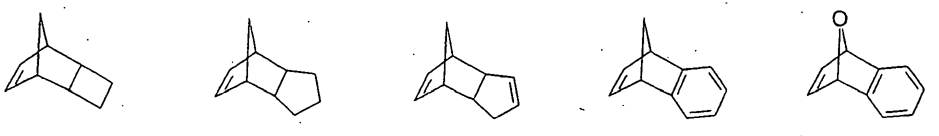

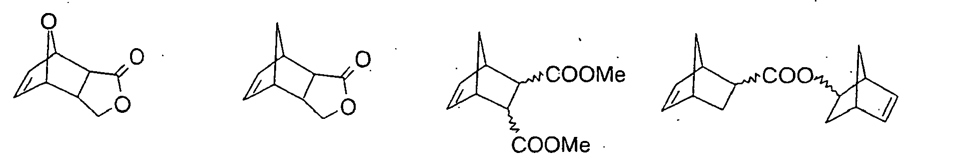

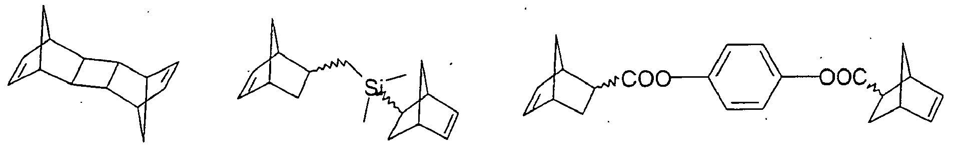

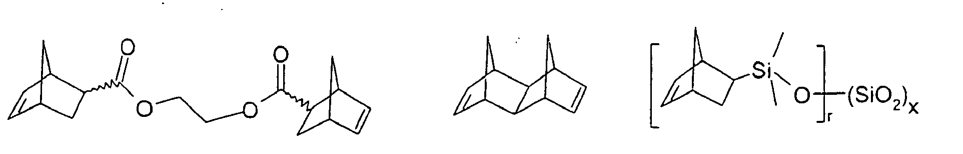

Darüber hinaus können oligomere Verbindungen mit metathesefähigen Gruppen als Komponente (a) verwendet werden. Hier sind insbesondere Oligomere von Norbornenderivaten, die neben der Norbornengruppe einen oligomeren Rest mit einer zahlenmittleren Molmasse 200 bis 10000 g/mol tragen, zu nennen. Die Norbornen-Reste können unsubstituiert oder durch C₁₋₁₀-Alkyl, Phenyl- und/oder C₁₋₅-Carbonsäure-C₁₋₁₀-alkylester-Gruppen substituiert sein.

Besonders bevorzugte Oligomere sind: 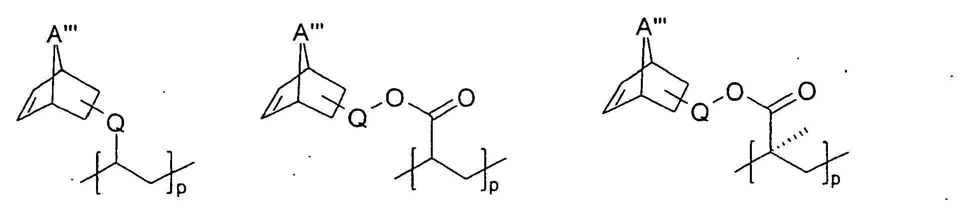 mit
- p =: 3 bis 100
- A''' =: O, CH₂, S, NH
- Q =: C₁-C₂₀-Alkylen, mit 0-3 Heteroatomen der Gruppe N, O, Si, P, S und 0-2 Carbonylgruppen als Spacer.

Die Anbindung von Q an den Norbornenrest kann über die Positionen 1, 4, 5, 6 oder 7 erfolgen.

Erfindungsgemäß fungiert der Füllstoff als Träger des Initiators und vorzugsweise auch als multifunktionelle und damit vernetzende Komponente, d.h. er kann über freie, nicht mit Initiator beladenen Ankergruppen in das Polymer bzw. Polymernetzwerk eingebaut werden. Bei der Verwendung vernetzender Füllstoffe und insbesondere bei der Verwendung vernetzender Füllstoffe und kovalent gebundener Initiatoren ergibt sich, daß sogar Materialien auf der Basis von Monomeren mit nur einer Norbornengruppe nach dem Härten praktisch keine bzw. nur geringe Anteile aufweisen, die mit geeigneten organischen Lösungsmitteln herausgelöst werden können.

Die Wahl des Lösungsmittels ist monomerabhängig, im Löseversuch sollte ein Lösungsmittel verwendet werden, in dem ein Homopolymer aus dem verwendeten Monomer löslich ist. Ein häufig anwendbares Lösungsmittel ist Dichlormethan. Die erfindungsgemäßen Zusammensetzungen enthalten nach dem Härten vorzugsweise weniger als 10 Gew.-%, bezogen auf die Gesamtmasse, an mit Dichlormethan herauslösbaren Anteilen.

Ein wesentliches Problem bei der Herstellung von Materialien, die durch RÖMP härtbar sind, liegt in der schlechten Löslichkeit der RÖMP-Initiatoren in den verschiedenen bekannten flüssigen Monomeren. Gemäß dem Stand der Technik werden die Initiatoren in der Zusammensetzung dispergiert. Das hat zur Folge, daß ein bedeutender Teil des eingesetzten Initiators, nämlich jener im Inneren der Initiatorpartikel, für das Monomer nicht zugänglich ist, folglich keine Polymerisation initiieren kann und somit nicht wirksam ist. Da es sich bei den Initiatoren meist um intensiv gefärbte Verbindungen handelt, können selbst im fertigen Formkörper mit dem freien Auge noch unumgesetzte Initiatorpartikel und Initiatoragglomerate erkannt werden.

Durch die erfindungsgemäße Fixierung des Initiators am Füllstoff kommt es zu einer quasi monomolekularen Verteilung des Initiators an der Oberfläche des Füllstoffs, und die gesamte eingesetzte Initiatormenge ist für das Monomer zugänglich. Durch die Trägerung des Initiators läßt sich die Initiatoreffizienz signifikant steigern oder anders gesagt, um das gleiche Resultat in den mechanischen Eigenschaften zu erzielen, muß weniger Initiator eingesetzt werden. Darüber hinaus wird eine bessere Verteilung des Initiators in der Zusammensetzung erzielt, so daß eine homogenere Härtung und damit gleichförmigere mechanische Eigenschaften erzielt werden.

Ein für Dentalanwendungen entscheidender Vorteil ist außerdem, daß sich durch die Bindung des Initiators an die Füllstoffoberfläche Verfärbungen des Materials durch den Initiator vermeiden lassen. Durch die Träger-Fixierung des Initiators können praktisch farblose und zahnfarbene Komposite hergestellt werden.

Den erfindungsgemäßen Dentalmaterialien können im Bedarfsfalle weitere Komponenten (d) zugesetzt werden, vor allem Stabilisatoren, UV-Absorber, Farbstoffe Pigmente und/oder Gleitmittel. Der Anteil dieser weiteren Komponenten liegt vorzugsweise jeweils im Bereich von 0 bis 1 Gew.-% und besonders bevorzugt 0 bis 0,2 Gew.-%.

Die erfindungsgemäßen Zusammensetzungen sind vorzugsweise lösungsmittelfrei. Unter Lösungsmittel werden hier bei Raumtemperatur flüssige Substanzen verstanden, die allein der Reaktionsführung oder der Erleichterung der Handhabung dienen, ohne in der fertig ausgehärteten Zusammensetzung noch vorhanden oder notwendig zu sein. Flüssige Monomere (a) sind demgemäß keine Lösungsmittel in diesem Sinne.

Die erfindungsgemässen Zusammensetzungen eignen sich besonders als Dentalwerkstoffe, insbesondere dentale Prothesenkunststoffe, zur Herstellung künstlicher Zähne und dentaler Restaurationen, als Verblendmaterialien oder Füllungsmaterialien.

Zur Herstellung beispielsweise von Prothesen oder künstlichen Zähnen werden die Zusammensetzungen in geeignete Polymerisationsformen überführt und dort ausgehärtet.

So können beispielsweise auf Basis thermisch aktivierbarer Initiatoren Prothesen, Zähne, Inlays oder Verblendmaterialien hergestellt werden, die sich extraoral bei 80 bis 100 °C aushärten lassen. Aufgrund der hydrophoben Eigenschaften der gebildeten Komposite zeichnen sich diese durch eine äußerst geringe Wasseraufnahme und Belagbildung aus. Dabei wird bei Materialien zur Herstellung von Zähnen und Prothesenmaterialien im Vergleich zu Inlay- oder Verblendmaterialien ein deutlich geringerer Füllstoffanteil verwendet. Für den Einsatz als direktes Füllungsmaterial eignen sich besonders erfindungsgemäße Zusammensetzungen mit hohem Füllstoffanteil auf der Basis von photochemisch aktivierbaren Initiatoren. Diese können mit thermisch aktivierbaren Initiatoren kombiniert werden, so daß durch photochemisch induzierte RÖMP zunächst eine gewisse Vorhärtung erzielt werden kann und anschließend durch eine länger dauernde thermisch induzierte RÖMP bei Körpertemperatur die Endhärtung stattfindet.

Zusammensetzungen zur Anwendung als Dentalwerkstoffe enthalten die einzelnen Komponenten vorzugsweise in den folgenden Anteilen:
(i) 10 bis 60 Gew.-%, bevorzugt 20 bis 40 Gew.-% und besonders bevorzugt 20 bis 40 Gew.-% mindestens eines Monomers/Oligomers, das der ringöffnenden Metathesepolymerisation zugänglich ist, und
(ii) 5 bis 90 Gew.-%, Füllstoff.

Bei der angegebenen Füllstoffmenge handelt es sich um die Gesamtmenge an Füllstoff, d.h. Füllstoff, der mit einem Initiator für die ringöffnende Metathespolymerisation beladen ist, und Füllstoff ohne Initiator. Füllstoff mit Initiator wird vorzugsweise in einer Menge von 5-90 Gew.-% verwendet, andere Füllstoffe werden vorzugsweise in einer Menge von 0 bis 85 Gew-% eingesetzt. Der Initiatorgehalt kann damit einerseits über die Menge des mit Initiator beladenen Füllstoffs und andererseits über die Beladung des Füllstoffs mit Initiator eingestellt werden. Erfindungsgemäß ist ein Initiatorgehalt im gesamten Komposit von 0,001-0,1 Gew.-%, insbesondere 0,01-0,08 Gew.-% bevorzugt.

Die Füllstoffmenge richtet sich nach der gewünschten Anwendung des Dentalwerkstoffs. Kompositzemente enthalten bevorzugt 40 bis 70 Gew.-% Füllstoff, Komposite, beispielsweise für Inlays oder zur Anwendung als Füllungsmaterial vorzugsweise 50 bis 85 Gew.-%. Materialien zur Herstellung von Zähnen und Prothesenmaterialien enthalten vorzugsweise 5 bis 30 Gew.-% Füllstoff.

Alle Prozentangaben beziehen sich, wenn nicht anders angegeben, auf die Gesamtmasse der Zusammensetzung.

Im Folgenden wird die Erfindung anhand von Ausführungsbeispielen näher erläutert:

### Beispiele

### Beispiel 1: Oberflächenmodifizierung von Füllstoff mit metathesefähigen Gruppen

### 1a: Oberflächenmodifizierung von pyrogenem SiO₂ mittels Silanisierung

4,0 g pyrogenes SiO₂ (Aerosil OX50 Degussa) wurden in einen Dreihalskolben eingewogen und ausgeheizt (standardgemäß bei 180-200°C, 5h, im Ölpumpenvakuum unter Rühren). Im N₂-Gegenstrom wurde ein ausgeheizter Rückflußkühler aufgesetzt und der Kolben mit einem Septum versehen. Der Rückflußkühler wurde an eine Thermostatisiereinheit angeschlossen, deren Temperatur auf 90°C eingestellt war, damit der im Zuge der Silanisierung abgespaltene Alkohol (Ethanol) nicht im Kühler, sondern erst in der darauf gesetzten wassergekühlten Destillationsbrücke kondensiert und so aus dem Gleichgewicht entfernt wird. Der Füllstoff wurde in 50 ml abs. Xylol suspendiert, 2,564 g (10 mmol) vom Silanisierungsagens Norborn-2-en-5-yltriethoxysilan zugesetzt und das Reaktionsgemisch 6h bei 140°C unter Stickstoff gerührt. Die abgekühlte Lösung wurde zentrifugiert, der silanisierte Füllstoff 3 Mal mit abs. Dichlormethan gewaschen und im Vakuum bei 40°C über Nacht getrocknet. Der Gehalt an Norbornenylgruppen konnte über C,H-Elementaranalyse (0,849% C und 0,200% H) mit 0,101 mmol/g Füllstoff bestimmt werden.

### 1b: Oberflächenmodifizierung eines Glasfüllstoffs mittels Silanisierung

Analog Beispiel **1a** wurden 4,0 g eines Barium-Silicatglases (GM27884, Schott Werke) ausgeheizt, in 50 ml abs. Xylol suspendiert und mit 2,564 g (10 mmol) des Silanisierungsmittels Norborn-2-en-5-yl-triethoxysilan umgesetzt und das Reaktionsgemisch 6h bei 140°C unter Stickstoff gerührt. Die abgekühlte Lösung wurde zentrifugiert, der silanisierte Füllstoff 3x mit je 50 ml abs. Dichlormethan gewaschen und im Vakuum bei 40°C über Nacht getrocknet. Der Gehalt an Norbornenylgruppen wurde über C,H-Elementaranalyse (0,244% C und 0,155% H) zu 0,029 mmol/g Füllstoff bestimmt.

### Beispiel 2: Immobilisierung von Initiator auf oberflächenmodifiziertem Füllstoff

### 2a: Immobilisierung des bei Raumtemperatur aktiven Metatheseinitiators Cl₂(PCy₃)₂Ru=CHPh

Die Immobilisierung des Metatheseinitiators Cl₂(PCy₃)₂Ru=CHPh wurde in einer Glovebox unter Stickstoffatmosphäre durchgeführt. 1,2 g des in Beispiel **1b** dargestellten, mit Norbornenylgruppen oberflächenmodifizierten Füllstoffs wurden in 2,86 ml einer Lösung von Cl₂(PCy₃)₂Ru=CHPh in abs. Dichlormethan der Konzentration 0,5 mg/ml suspendiert und eine Stunde bei Raumtemperatur gerührt. Der Überstand der Suspension war farblos. Ca. 6% der Norbornenylgruppen des oberflächenmodifiziertem Füllstoffes waren mit Initiator kovalent beladen. Der mit Initiator beladene Reaktivfüllstoff wurde vom Lösungsmittel abzentrifugiert, 2 Stunden am Ölpumpenvakuum getrocknet und anschließend tiefgekühlt gelagert.

### 2b: Immobilisierung des thermisch aktivierbaren Initiators RuCl₂(CHCH₂CH₂-C,N-2-C₅H₄N) (PiPr₃)

0,608 g des in Beispiel **1b** dargestellten, mit Norbornenylgruppen oberflächenmodifizierten Füllstoffs wurden in einer Lösung von 1,8 mg (0,0029 mmol) RuCl₂(CHCH₂CH₂-C,N-2-C₅H₄N) (PiPr₃) in 3 ml abs. Dichlormethan suspendiert. Das Lösungsmittel wurde an einem Rotationsverdampfer abgezogen. Der Initiator lag adsorptiv gebunden an der Füllstoffaberfläche vor. Die Beladung des Füllstoffs mit Initiator betrug 0,0048 mmol Initiator/g Füllstoff.

### Beispiel 3: Polymerisation und Herstellen von Kompositformteilen

### 3a: RÖMP von endo,exo-(Bicyclo[2.2.1]hept-5-en)-2-carbonsäuremethylester (BCHCM) in Gegenwart eines bei Raumtemperatur aktiven Initiators (Cl₂(PCy₃)₂Ru=CHPh)

320 mg des in Beispiel **2a** beschriebenen, initiierenden Füllerstoffs sowie 280 mg des in Beispiel **1b** beschriebenen oberflächenmodifizierten Füllstoffs wurden mit 320 mg **BCHCM** bei Raumtemperatur innig vermischt (Füllgrad: 65,2 Gew.-%, Monomer/Initiator-Verhältnis = 4530) und sofort in Biegefestigkeitsprüfformen (25 x 2 x 2 mm) überführt. Die Aushärtung erfolgte bei 80°C über eine Zeit von 20 h. Das ausgehärtete Material zeigte einen Biege-E-Modul von 5780 MPa und war cremefarben. Die Extraktion des Formkörpers mit 7,5 g Dichlormethan (Raumtemperatur, 5 Tage), das ein Lösungsmittel für Poly(BCHCM) ist, zeigte, daß die Probekörper formstabil blieben. Es ließen sich 0,42 Gew.-% an unumgesetzten Monomer (bezogen die eingesetzte Monomermenge), sowie 9,13 Gew.-% an Polymer (Mₙ = 5390 g/mol, M_{w} = 16600 g/mol, PDI = 3,1), bezogen auf das Gesamtgewicht (das sind 26,24 Gew-% bezogen auf die eingesetzte Monomermenge), extrahieren.

Es wurden weitere Versuche durchgeführt (vgl.Tabelle 1), die zeigten, daß der Initiatorgehalt durch Zumischen eines Reaktivfüllstoffs nach Beispiel **1b** abgesenkt werden kann, ohne daß sich die mechanischen Eigenschaften dadurch verschlechtern, wobei jedoch zunehmend "farblosere" Produkte erhalten werden konnten. Der Initiator wurde analog zu Beispiel 2a mit den in Tabelle 1 angegebenen Initiatormengen beladen.

**Tabelle 1 Eigenschaften von RÖMP-Produkten mit bei Raumtemperatur aktiven Initiatoren**

| **Füller nach 2a** **[mg]** | **immob. Initiator** **[mg/gFS]**¹ | **Füller nach 1b** **[mg]** | **Monomer** **[mg]** | **Initiatorgehalt**² **[Gew%]** | **Füll grad** **[%]** | **Monomer/Initiator**³ | **E-Modul** **[Mpa]**⁴ | **Farbe** |
|---|---|---|---|---|---|---|---|---|
| 393 | 2,16 | 0 | 391 | 0,108 | 50,1 | 2487 | 3450 | karamell |
| 362 | 2,03 | 40 | 405 | 0,091 | 49,8 | 2983 | 3570 | karamell hell |
| 307 | 2,03 | 91 | 403 | 0,078 | 49,7 | 3501 | 2970 | karamell hell |
| 262 | 2,03 | 144 | 406 | 0,066 | 50,0 | 4132 | 3260 | karamell hell |
| 404 | 0,9 | 0 | 405 | 0,045 | 49,9 | 6015 | 3860 | cremefarben |
| 262 | 0,9 | 40 | 301 | 0,039 | 50,1 | 6893 | 3430 | cremefarben |
| 288 | 0,9 | 112 | 400 | 0,032 | 50,0 | 8333 | 3220 | cremefarben |

| | | | | | | | | |
|---|---|---|---|---|---|---|---|---|
| ¹ auf Füllstoff immobilisierter Metatheseinitiator in mg Initiator pro Gramm Füllstoff (FS) nach 2a | | | | | | | | |
| ² Initiatorgehalt bezogen auf das Gesamtgewicht des Composits | | | | | | | | |
| ³ Molverhältnis Monomer/Initiator | | | | | | | | |
| ⁴ Biege-E-Modul ermittelt nach EN 24 049 / ISO 4049 | | | | | | | | |

### 3b: RÖMP von BCHCM in Gegenwart eines physikalisch gebundenen Metatheseinitiators (Cl₂(PCy₃)₂Ru=CHPh)

1,0 g eines mit γ-Methacryloxypropyltrimethoxysilan oberflächenodifizierten Glasfüllstoffs (GM27884, Schott Werke) wurden in einer Lösung von 0,64 mg Cl₂(PCy₃)₂Ru=CHPh in 1 ml abs. Dichlormethan suspendiert. Das Lösungsmittel wurde im Vakuum abgezogen. 601 mg dieses mit Metatheseinitiator beladenen, inerten Füllstoffs wurden mit 320 mg **BCHCM** bei Raumtemperatur innig vermischt (Füllgrad: 65,3%, Monomer/Initiator-Verhältnis = 4499) und sofort in Biegefestigkeitsprüfformen überführt.

Das analog zu **3a** ausgehärtete Material zeigte einen Biege-E-Modul von 5440 MPa und war cremefarben. Extrahierte man den Formkörper in 7,5 g Dichlormethan (Raumtemperatur, 5 Tage), so löste sich dieser vollständig auf, der Füllstoff sank zu Boden, die überstehende Lösung war vom Initiator leicht violettbräunlich gefärbt. Der gelöste Poly(**BCHCM**) konnte in Methanol ausgefällt werden.

### 3c: RÖMP von BCHCM in Gegenwart eines nicht füllstoffgebundenen Initiators (Vergleichsbeispiel)

Der Metatheseinitiätor wurde auf herkömmlicher Weise der zu polymerisierenden Masse zugemischt. Auf ein Uhrglas wurden 0,19 mg Cl₂(PCy₃)₂Ru=CHPh eingewogen. Zur Erzielung einer feinen Verteilung des Initiators wurde die entsprechende Menge einer Lösung des Metatheseinitiators in Dichlormethan (0,5 mg / ml) aufgetragen und das Lösungsmittel langsam verdampfen gelassen. 300 mg des in Beispiel **1b** beschriebenen oberflächenmodifizierten Füllers und 160 mg **BCHCM** wurden mit dem Initiator innig vermischt (Füllgrad 65,2%, Monomer/Initiator = 4499). Das analog zu **3a** ausgehärtete Material zeigte einen Biege-E-Modul von 5030 MPa. In Tabelle 2 sind Ergebnisse weiterer Versuche aufgeführt, die zeigen, daß bereits mit einem Monomer/Initiator-Verhältnis von 6200 (Initiatorgehalt im Composit: 0,044 Gew%) keine Formkörper mehr hergestellt werden können. Grund dafür ist die schlechte Löslichkeit des Initiators im flüssigen BCHCM. Der Initiator konnte nicht fein genug verteilt werden, es lagen Agglomerate der Initiatormoleküle vor, von denen nur die Moleküle an der Oberfläche eine Polymerisation initiieren können. Somit war die effektiv wirksame Initiatormenge zu gering, um die Dentalmasse auszuhärten.

Gemäß den in Tabelle 1 gezeigten Ergebnissen konnten die erfindungsgemäßen Werkstoffe noch bei einem Mönomer/Initiator-Verhältnis von 8333 (entspricht einem Initiatorgehalt von 0,03 Gew.-%) zu cremefarbenen Formkörpern gehärtet werden. Die erfindungsgemäßen Zusammensetzungen wiesen zudem mechanische Eigenschaften auf, die bei herkömmlichen Materialien erst mit sehr viel höheren Initiatormengen, d.h. bei geringerem Monomer zu Initiator-Verhältnis erzielt werden.

In analog durchgeführten Versuchen konnten Formkörper mit vergleichbaren mechanische Eigenschaften mit einem Initiatorgehalt von 0,02 Gew.-% hergestellt werden.

**Tabelle 2**

| Eigenschaften von RÖMP-Produkten mit herkömmlichen Initiatoren | | | | | | | |
|---|---|---|---|---|---|---|---|
| **Initiator** **[mg]**¹ | **Füller nach 1b** **[mg]** | **Monomer** **[mg]** | **Initiatorgehalt**² **[Gew%]** | **Füllgrad** **[%]** | **Monomer/Initiator**³ | **E-Modul**⁴ **[Mpa]** | **Farbe** |
| 0,36 | 202 | 208 | 0,088 | 49,27 | 3124 | 3480 | beige |
| 0,18 | 201 | 208 | 0,044 | 49,14 | 6249 | -⁵ | - |

| | | | | | | | |
|---|---|---|---|---|---|---|---|
| ¹ mg an zugemischtem Initiator **1** | | | | | | | |
| ² Initiatorgehalt bezogen auf das Gesamtgewicht des Composits | | | | | | | |
| ³ Molverhältnis Monomer/Initiator | | | | | | | |
| ⁴ Biege-E-Modul ermittelt nach EN 24 049 / ISO 4049 | | | | | | | |
| ⁵ Die Mischung dieser Zusammensetzung härtete nicht aus, daher konnten keine Formkörper erhalten werden. | | | | | | | |

### 3d: RÖMP von BCHCM in Gegenwart eines thermisch schaltbaren Metatheseinitiators (RuCl₂(=CHCH₂CH₂-C,N-2-C₅H₄N) (PiPr₃))

183,5 mg des in Beispiel **2b** hergestellten, mit dem thermisch schaltbaren Metatheseinitiator beladenen Reaktivfüllers wurden mit 184,8 mg **BCHCM** bei Raumtemperatur innig vermischt (Füllgrad 49,8%, Monomer/Initiatorverhältnis = 1370) und sofort in eine Biegefestigkeitsprüfform überführt. Durch Temperaturerhöhung auf 100°C wurde der Initiator aktiviert und die Metatheseplymerisation gestartet. Die Reaktionsdauer betrug 2h. Das ausgehärtete Material zeigte einen Biege-E-Modul von 4370 MPa. Durch teilweise Substitution des initiierenden Füllstoffs durch den oberflächenmodifiziierten Reaktivfüllstoff ließ sich der effektive Initiatorgehalt der Komposite unter Beibehaltung bzw. unter Verbesserung der mechanischen Eigenschaften schrittweise verringern (vgl. Tabelle 3). Mit höherem Füllgrad sowie mit sinkendem Initiatorgehalt wurden die Proben zunehmend "farbloser".

**Tabelle 3**

| Eingenschaften von RÖMP-Produkten mit thermisch aktivierbaren Initiatoren | | | | | | | | |
|---|---|---|---|---|---|---|---|---|
| **Füller nach 2b** **[mg]** | **Initiator** **[mg/gFS]**¹ | **Füller nach 1b** **[mg]** | **Monomer** **[mg]** | **Initiatorgehalt** **[Gew%]**² | **Füllgrad** **[%]** | **Monomer/Initiator**³ | **E-Modul** **[MPa]**⁴ | **Farbe** |
| 91 | 2,96 | 110 | 209 | 0,066 | 49,02 | 3133 | 3830 | karamell |
| 37 | 2,96 | 166 | 202 | 0,027 | 51,54 | 7448 | 4370 | creme |

| | | | | | | | | |
|---|---|---|---|---|---|---|---|---|
| ¹ Initiatormenge in mg adsorbiert pro g Füller (FS) nach 2b | | | | | | | | |
| ² Initiatorgehalt bezogen auf das Gesamtgewicht des Composits | | | | | | | | |
| ³ Molverhältnis Monomer/Initiator | | | | | | | | |
| ⁴ Biege-E-Modul ermittelt nach EN 24 049 / ISO 4049 | | | | | | | | |

## Patentansprüche

1. Verwendung einer Zusammensetzung enthaltend
(a) mindestens ein Monomer und/oder Oligomer, das der ringöffnenden Metathesepolymerisation zugänglich ist,
(b) mindestens einen Füllstoff, und
(c) mindestens einen Initiator für die ringöffnende Metathespolymerisation,
**dadurch gekennzeichnet, daß** der Initiator mittels eines Bindemittels chemisch oder physikalisch an den Füllstoff gebunden ist, zur Herstellung eines Dentalwerkstoffs.

2. Verwendung nach Anspruch 1, wobei der Initiator mittels eines Silans der Formel (1) chemisch an den Füllstoff gebunden ist:
[(G-X)ᵣ-R¹-Y-R²]ₓ-SiR³_{y}R⁴_{z} Formel 1
wobei die Variablen der Formel (1) die folgenden Bedeutungen haben:
G = ein metathesefähiger, linearer oder verzweigter organischer Rest mit m Kohlenstoffatomen, wobei m eine ganze Zahl von 1 bis 40 ist, und 0 bis (m-2) Heteroatomen, ausgewählt aus N, O, Si, P und S, oder
ein metathesefähiger, cyclischer oder polycyclischer, cycloaliphatischer oder aromatischer organischer Rest mit m' Kohlenstoffatomen, wobei m' eine ganze Zahl von 3 bis 63 ist, und 0 bis (m'-2) Heteroatomen, ausgewählt aus N, O, Si, P und S, ist.
X, Y = unabhängig voneinander -(C(=O)-O-, -C(=O)-N-, -O-C(=O)-O-, -O-C(=O)-NR⁵-, -CR⁵=N-, -O-, -S-, mit R⁵= H, C₁-C₆-Alkyl, Benzyl oder entfällt;
R¹ = ein (r+1)-wertiger, linearer oder verzweigter aliphatischer, cycloaliphatischer oder aromatischer organischer Rest oder entfällt;
R² = eine C₁-C₅-Alkylengruppe oder entfällt;
R³ = Halogen, Hydroxy, eine C₁-C₅-Alkoxy oder C₁-C₅-Alkoxy- oder C₁-C₅-Acyloxygruppen;
R⁴ = C₁-C₁₂-Alkyl, C₃-C₁₂-Cycloalkyl oder Phenyl;
r = 1, 2 oder 3;
x = 1, 2 oder 3;
y = 1, 2 oder 3;
z = 0, 1 oder 2;
wobei die Summe x+y+z gleich 4 ist.

3. Verwendung nach Anspruch 2, wobei die Variablen der Formel (1) die folgende Bedeutung haben:
G = ein Rest der Formel (2) 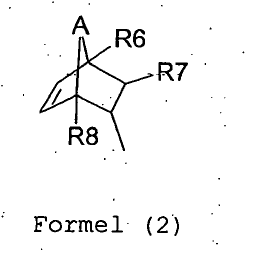 in der die Variablen wie folgt definiert sind:
A = 0, NH, S, ein gesättigter oder ungesättigter organischer Rest mit t Kohlenstoffatomen, wobei t eine ganze zahl von 1 bis 12. ist, der 0 bis (t-1) Heteroatome, ausgewählt aus N, O, Si, P und S, enthalten kann;
R⁶, R⁷, R⁸ = unabhängig von einander jeweils ein linearer oder verzweigter aliphatischer Rest mit q Kohlenstoffatomen, wobei q eine ganze Zahl von 1 bis 15 ist, ein cyclischer oder polycyclischer organischer Rest mit q' Kohlenstoffatomen, wobei q' eine ganze Zahl von 3 bis 15 ist, und 0 bis (q-1) bzw. 0 bis (q'-1) Heteroatomen, ausgewählt aus N, O, Si, P, und S;
X,Y = entfällt;
R¹ = ein linearer C₁- bis C₅-Alkylrest oder entfällt;
R²= entfällt;
R³ = Chlor, Methoxy, Ethoxy;
R⁴ = Methyl;
r = 1;
x = 1;
y = 1, 2 oder 3;
z = 0, 1 oder 2.

4. Verwendung nach Anspruch 1, wobei der Initiator mittels eines Silans der Formel (3) physikalisch an den Füllstoff gebunden ist:
(R⁹)ₐ(R¹⁰)_{b}(R¹¹)_{c} Si(R¹²)_{d} Formel (3)
R¹²: Halogen, Hydroxy, eine C₁-C₅-Alkoxy- oder C₁-C₅-Acyloxygruppe
R⁹, R¹⁰, R¹¹: unabhängig voneinander ein gesättigter oder ungesättigter, linearer oder verzweigter organischer Rest mit s Kohlenstoffatomen, wobei s eine.ganze Zahl von 2 bis 20 ist, und 0 bis (s-1) Heteroatomen, ausgewählt aus N, 0, Si, P und S ; oder ein gesättigter, ungesättigter oder aromatischer, cyclischer oder polycyclischer organischer Rest mit s' Kohlenstoffatomen, wobei s' eine ganze zahl von 3 bis 15 ist, mit 0 bis (s'-1) Heteroatomen, ausgewählt aus N, O, Si, P und S;
a = 0, 1, 2 oder 3;
b = 0, 1, 2 oder 3;
c = 0, 1, 2 oder 3;
d = 1, 2 oder 3,
wobei die Summe aus a+b+c+d 4 ist.

5. Verwendung nach einem der Ansprüche 1 bis 4, wobei der Initiator ein bei Raumtemperatur aktiver Initiator ist.

6. Verwendung nach Anspruch 5, wobei der Initiator eine Verbindung der Formel (4) ist: 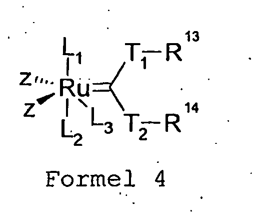
Z = Cl, Br, F, I, Tosylat oder eine der für L₁, L₂, L₃ angegebenen Bedeutungen;
L₁, L₂, L₃ = unabhängig voneinander P(R¹⁵)₃ mit R¹⁵ = Phenyl, Isopropyl, Cyclohexyl oder 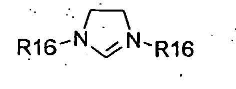 bzw. 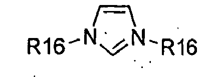 mit R¹⁶ = Mesityl (2,4,6-Trimethylphenyl; MES) ; oder
Pyridin, das unsubstituiert oder in Position 2 bzw in Position 2 und 4 mit Br, Cl, F, I, OCH₃ substituiert ist; oder
entfällt;
R₁₃, R₁₄ = voneinander unabhängig H oder ein aromatischer oder aliphatischer, polycyclischer oder kondensierter C₆-C₂₀-Ring bzw. Ringsystem, mit 0-5 Heteroatomen im Ring, ausgewählt aus N, S, O, P, wobei der Ring bzw. das Ringsystem unsubstituiert ist oder zusätzlich mit 0-5 Substituenten des Typs -Cl, -Br, -I, -F, -OR¹⁷, -CH=N-R¹⁷, -C(=O)R¹⁷, -C(=O)OR¹⁷, -OC(=O)R¹⁷ substituiert sein kann, wobei R¹⁷ ein lineares oder verzweigtes, acyclisches C₁-C₁₉-Alkyl oder cyclisches C₃-C₁₉-Alkyl oder ein aromatischer Rest mit 6 bis 14 Kohlenstoffatomen ist, wobei R¹⁷ 0 bis 5 Heteroatomen, ausgewählt aus N, O, Si, S enthalten kann;
T₁, T₂ = unabhängig voneinander -O-, -S-, oder ein gesättigtes oder ungesättigtes C₁ bis C₄-Alkylen, welches zusätzlich 0-2 Heteroatome der Gruppe N, O*,* S, Si beinhalten kann oder entfällt.

7. Verwendung nach einem der Ansprüche 1 bis 5, wobei der Initiator ein thermisch oder photochemisch aktivierbarer Initiator ist.

8. Verwendung nach Anspruch 7, wobei der Initiator 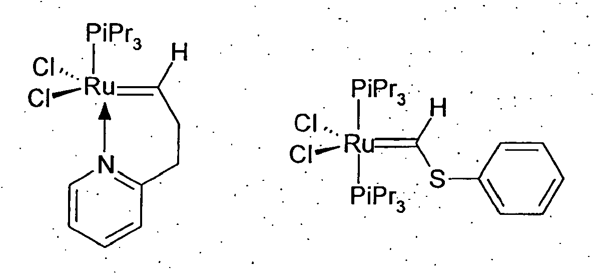 oder eine Verbindung der Formel (5) ist: 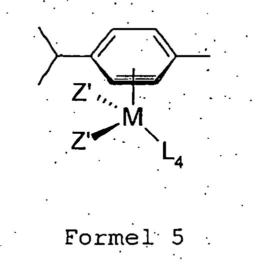
M = Os oder Ru;
Z' = Cl, Br, F, I, Tosylat, oder eine der für L₄ angegebenen Bedeutungen;
L₄ = P (R¹⁸) 3 mit R¹⁸= Phenyl, Isopropyl, Cyclohexyl oder 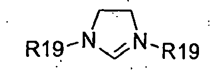 bzw. 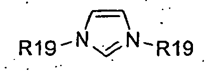 mit R¹⁹= Mesityl (2,4,6-Trimethylphenyl).

9. Verwendung nach einem der Ansprüche 1 bis 8, wobei der Füllstoff mit 0,00001 bis 0,1 mmol Initiator pro Gramm Füllstoff beladen ist.

10. Verwendung nach einem der Ansprüche 1 bis 9, wobei die Zusammensetzung eine zweite Füllstoffkomponente enthält, die mit einem Silan der Formel (1) oberflächenmodifiziert ist.

11. Verwendung nach einem der Ansprüche 1 bis 10, wobei die Zusammensetzung als Komponente (a) mindestens eine bi- oder multicyclische Ringverbindung mit mindestens einer endocyclischen Doppelbindung enthält.

12. Verwendung nach Anspruch. 11, wobei die Zusammensetzung einen carbocyclischen oder heterocyclischen Bicyclo[x'.y'.z']kohlenwasserstoff enthält, wobei x', y' und z' unabhängig voneinander jeweils eine ganze Zahl von 1 bis 6 sind.

13. Verwendung nach Anspruch 12, wobei die Zusammensetzung ein Norbornenderivat der Formel (6) und/oder (7) enthält 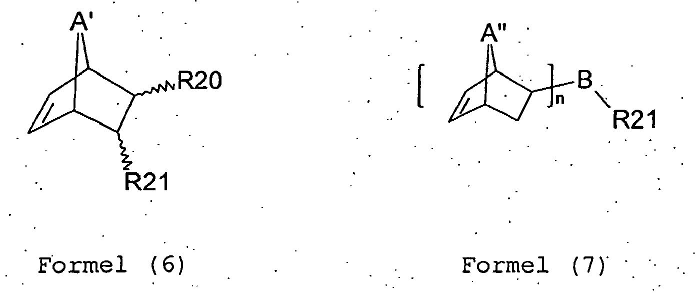 in denen die Variablen die folgenden Bedeutungen haben:
A' , A" = O, S, NH, ein gesättigter oder ungesättigter organischer Rest mit u Kohlenstöffatömen, wobei u eine ganze Zahl von 1 bis 15 ist, der 0 bis (u-1) Heteroatome, ausgewählt aus N, O, Si, P und S, enthalten kann;
B = -CH₂- oder entfällt;
R²⁰, R²¹ = H oder ein gesättigter oder ungesättigter organischer Rest, mit v Kohlenstoffatomen, wobei v eine ganze Zahl von 1 bis, 30 ist, und mit 0 bis (v-1) Heteroatomen aus der Gruppe N, O, Si, P, S und F; oder
R²⁰ und R²¹ bilden zusammen mit den Atomen, an die sie gebunden sind, ein ankondensiertes, gesättigtes oder ungesättigtes alicyclisches Ring- system mit 4 bis 12 Kohlenstoffatomen oder ein ankondensiertes aromatisches Ringsystem mit 6 bis 12 Kohlenstoffatomen, das seinerseits durch C₁₋₆-Alkyl oder Benzyl substituiert sein kann;
R²² = n-fach substituiertes C₁- bis C₁₀-Alkylen, C₆-bis C₁₀-Arylen, -O-C(=O)-Phehylen-C(=O)-O-, (-C(=O)-)₄(C₆H₂),2,4,6-Trioxo-1, 3, 5-triazinyl, -O-C(=O)-(CH₂)ₘ-C(=O)-O-; -O-C(=Q)-NH-(CH₂)ₘ-NH-C(=O)-O-, wobei m eine ganze Zahl von 1 bis 10 ist;
n = eine ganze Zahl von 1 bis 4.

14. Verwendung nach einem der Ansprüche 1 bis 13, wobei der Initiator an einen anorganischen partikulären Füllstoff gebunden ist.

15. Verwendung nach Anspruch 14, wobei der partikuläre Füllstoff ein Füllstoff mit einer primären Partikelgröße von 5 bis 500 nm ist.

16. Verwendung nach Anspruch 14 oder 15, wobei der partikuläre Füllstoff pyrogene Kieselsäure, Fällungskieselsäure, ein Mischoxid aus SiO₂, ZrO₂, TiO₂, TaO₂, La₂O₃, Yb₂O₃ und/oder CeO₂ enthält.

17. Verwendung nach Anspruch 14, wobei der partikuläre Füllstoff eine durchschnittlichen Teilchengröße von 0,01 bis 5 µm aufweist.

18. Verwendung nach Anspruch 17, wobei der partikuläre Füllstoff Quarz-, Glaskeramik- oder Glaspulver enthält.

19. Verwendung nach einem der Ansprüche 1 bis 18, wobei die Zusammensetzung als Füllstoff Ytterbiumtrifluorid enthält.

20. Verwendung nach einem der Ansprüche 1 bis. 19, wobei die Zusammensetzung weiterhin
(d) mindestens ein Additiv aus der Gruppe Stabilisator, UV-Absorber, Farbstoff, Pigment, Gleitmittel enthält.

21. Verwendung nach einem der Ansprüche 1 bis 20, wobei die Zusammensetzung lösungsmittelfrei ist.

22. Verwendung nach einem der Ansprüche 1 bis 21, wobei die Zusammensetzung
(i) 10 bis 60 Gew.-% Monomer/Oligomer
(ii) 5 bis 90 Gew.-% Füllstoff
enthält.

23. Verwendung nach einem der Ansprüche 1 bis 22, wobei die Zusammensetzung
0,001 bis 0,1 Gew.-% Initiator enthält.

24. Verwendung eines Kits, der das Monomer/Oligomer (a) und initiatorhaltigen Füllstoff gemäß einem der Ansprüche 1 bis 23 in räumlich getrennter Form enthält, zur Herstellung eines Dentalwerkstoffs.

25. Verwendung nach einem der Ansprüche 1 bis 24 zur Herstellung von Prothesenkunststoff, künstlichen Zähnen, Verblendmaterial oder Füllmaterial.

## Claims

1. Use of a composition comprising
(a) at least one monomer and/or oligomer which is available for ring-opening metathesis polymerisation,
(b) at least one filler, and
(c) at least one initiator for ring-opening metathesis polymerisation, **characterised in that** the initiator is chemically or physically bound to the filler by means of a binder, and said composition is used for manufacturing a dental material.

2. Use according to claim 1, wherein the initiator is chemically bound to the filler by means of a silane of formula (1):
[(G-X)ᵣ-R¹-Y-R²]ₓ-SiR³_{y}R⁴_{z} Formula 1
wherein the variables of formula (1) have the following meanings:
G = a linear or branched organic group capable of metathesis with m carbon atoms, m being an integer from 1 to 40, and 0 to (m-2) heteroatoms, selected from N, O, Si, P and S, or
a cyclic or polycyclic, cycloaliphatic or aromatic organic group capable of metathesis containing m' carbon atoms, m' being an integer from 3 to 63, and 0 to (m'-2) heteroatoms, selected from N, O, Si, P and S.
X, Y = independently of each other -C(=O)-O-, -C(=O)-N-, -O-C(=O)-O-, -O-C(-O)-NR⁵ -, -CR⁵=N-, -O-, -S-, with R⁵ = H, C₁-C₆ alkyl, benzyl or is absent;
R¹ = an (r+1)-valent, linear or branched aliphatic, cycloaliphatic or aromatic organic group or is absent;
R² = a C₁-C₅ alkylene group or is absent;
R³ = halogen, hydroxy, a C₁-C₅ alkoxy- or C₁-C₅ acyloxy group;
R⁴ = C₁-C₁₂ alkyl, C₃-C₁₂ cycloalkyl or phenyl;
r = 1,2 or 3;
x= 1, 2 or 3;
y = 1, 2 or 3;
z = 0, 1 or 2,
wherein the sum x+y+z is equal to 4.

3. Use according to claim 2, wherein the variables of formula (1) have the following meaning:
G = a group of formula (2) 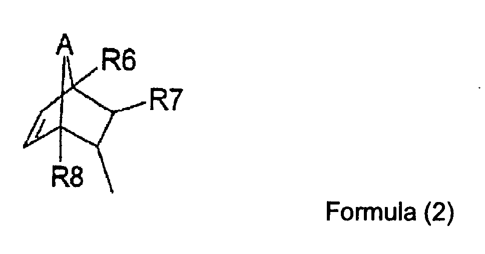
in which the variables are defined as follows:
A = O, NH, S, a saturated or unsaturated organic group with t carbon atoms, t being an integer from 1 to 12, which can comprise 0 to (t-1) heteroatoms, selected from N, O, Si, P and S;
R⁶, R⁷, R⁸ = independently of each other in each case a linear or branched aliphatic group containing q carbon atoms, q being an integer from 1 to 15, a cyclic or polycyclic organic group with q' carbon atoms, q' being an integer from 3 to 15, and 0 to (q-1) or 0 to (q'-1) heteroatoms, selected from N, O, Si, P, and S;
X, Y = is absent;
R¹ = a linear C₁- to C₅ alkyl group or is absent;
R² = is absent;
R³ = chlorine, methoxy, ethoxy;
R⁴ = methyl;
r = 1;
x = 1;
y= 1, 2 or 3;
z= 0, 1 or 2.

4. Use according to claim 1, wherein the initiator is physically bound to the filler by a silane of formula (3):
(R⁹)ₐ(R¹⁰)_{b}(R¹¹)_{c}Si(R¹²)_{d} Formula (3)
R¹²: halogen, hydroxy, a C₁-C₅ alkoxy- or C₁-C₅ acyloxy group;
R⁹, R¹⁰, R¹¹: independently of each other a saturated or unsaturated, linear or branched organic group containing s carbon atoms, s being an integer from 2 to 20, and 0 to (s-1) heteroatoms, selected from N, O, Si, P and S; or
a saturated, unsaturated or aromatic, cyclic or polycyclic organic group with s' carbon atoms, s' being an integer from 3 to 15, containing 0 to (s'-1) heteroatoms, selected from N, O, Si, P and S;
a = 0, 1, 2 or 3;
b = 0, 1, 2 or 3;
c= 0, 1, 2 or 3;
d = 1, 2 or 3,
wherein the sum of a+b+c+d is 4.

5. Use according to one of claims 1 to 4, wherein the initiator is an initiator active at room temperature.

6. Use according to claim 5, wherein the initiator is a compound of formula (4): 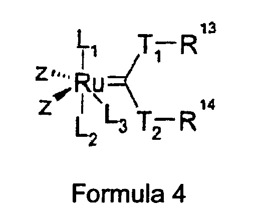
Z = Cl, Br, F, I, tosylate or one of the meanings given for L₁, L₂, L₃;
L₁, L₂, L₃ = independently of each other P(R¹⁵)₃ with R¹⁵ = phenyl, isopropyl, cyclohexyl or 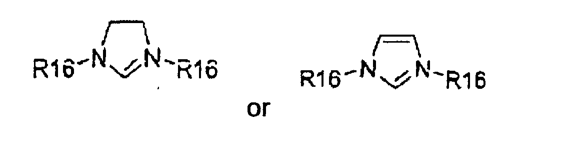 with R¹⁶ = mesityl (2,4,6-trimethylphenyl; MES); or pyridine which is unsubstituted or substituted in position 2 or in positions 2 and 4 with Br, Cl, F, I, OCH₃; or is absent;
R¹³, R¹⁴ = independently of each other H or an aromatic or aliphatic, polycyclic or condensed C₆-C₂₀ ring or ring system, containing 0-5 heteroatoms in the ring, selected from N, S, O, P, wherein the ring or the ring system is unsubstituted or can be further substituted with 0-5 substituents of the type -Cl, -Br, -l, -F, -OR¹⁷, -CH=N-R¹⁷, -C(=O)R¹⁷, -C(=O)OR¹⁷, -OC(=O)R¹⁷, wherein R¹⁷ is a linear or branched, acyclic C₁-C₁₄ alkyl or cyclic C₃-C₁₄ alkyl or an aromatic group containing 6 to 14 carbon atoms, wherein R¹⁷ can comprise 0 to 5 heteroatoms, selected from N, O, Si, S;
T₁, T₂ = independently of each other -O-, -S-, or a saturated or unsaturated C₁ to C₄ alkylene that can additionally contain 0-2 heteroatoms of the group N, O, S, Si or is absent.

7. Use according to one of claims 1 to 5, wherein the initiator is a thermally or photochemically activatable initiator.

8. Use according to claim 7, wherein the initiator is 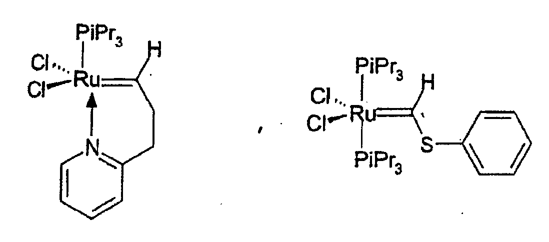 or a compound of formula (5): 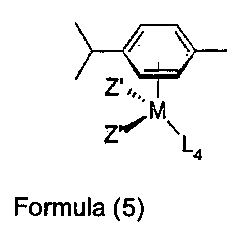
M = Os or Ru;
Z' = Cl, Br, F, l, tosylate or one of the meanings given for L₄;
L₄ = P(R¹⁸)₃ with R¹⁸ = phenyl, isopropyl, cyclohexyl or 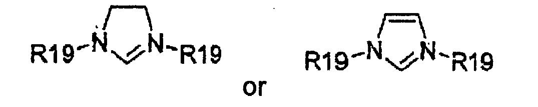 with R¹⁹ =mesityl (2,4,6-trimethylphenyl).

9. Use according to one of claims 1 to 8, wherein the filler is loaded with 0.00001 to 0.1 mmol initiator per gram of filler.

10. Use according to one of claims 1 to 9, wherein the composition comprises a second filler component that is surface-modified with a silane of formula (1).

11. Use according to one of claims 1 to 10, wherein the composition comprises at least one bi- or multicyclic ring compound with at least one endocyclic double bond as component (a).

12. Use according to claim 11, wherein the composition comprises a carbocyclic or heterocyclic bicyclo[x'.y'.z']hydrocarbon, wherein x', y' and z' independently of each other are each an integer from 1 to 6.

13. Use according to claim 12, wherein the composition comprises a norbornene derivative of formula (6) and/or (7) 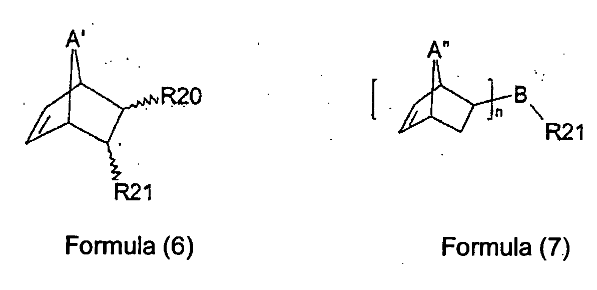 in which the variables have the following meanings:
A', A" = O, S, NH, a saturated or unsaturated organic group containing u carbon atoms, u being an integer from 1 to 15, which can contain 0 to (u-1) heteroatoms, selected from N, O, Si, P and S;
B = -CH₂- or is absent;
R²⁰, R²¹ = H or a saturated or unsaturated organic group containing v carbon atoms, v being an integer from 1 to 30, and containing 0 to (v-1) heteroatoms from the group N, O, Si, P, S and F; or
R²⁰ and R²¹ together with the atoms to which they are bonded form a fused, saturated or unsaturated alicyclic ring system containing 4 to 12 carbon atoms or a fused aromatic ring system containing 6 to 12 carbon atoms, which itself can be substituted by C₁-C₆ alkyl or benzyl;
R²² = n-times substituted C₁- to C₁₀ alkylene, C₆- to C₁₀ arylene, -O-C(=O)-phenylene-C(=O)-O-, (-C(=O)-)₄(C₆H₂) 2,4,6-trioxo-1,3,5-triazinyl, -O-C(=O)-(CH₂)ₘ-C(=O)-O-; -O-C(=O)-NH-(CH₂)ₘ-NH-C(=O)-O-, wherein m is an integer from 1 to 10;
n = an integer from 1 to 4.

14. Use according to one of claims 1 to 13, wherein the initiator is bound to an inorganic particulate filler.

15. Use according to claim 14, wherein the particulate filler is a filler with a primary particle size of 5 to 500 nm.

16. Use according to claim 14 or 15, wherein the particulate filler comprises pyrogenic silicic acid, precipitated silicic acid, a mixed oxide of SiO₂, ZrO₂, TiO₂, TaO₂, La₂O₃, Yb₂O₃ and/or CeO₂.

17. Use according to claim 14, wherein the particulate filler has an average particle size of 0.01 to 5 µm.

18. Use according to claim 17, wherein the particulate filler contains quartz powder, glass ceramic powder or glass powder.

19. Use according to one of claims 1 to 18, wherein the composition comprises ytterbium trifluoride as the filler.

20. Use according to one of claims 1 to 19, wherein the composition further comprises
(d) at least one additive from the group stabiliser, UV-absorber, dye, pigment, lubricant.

21. Use according to one of claims 1 to 20, wherein the composition is solvent-free.

22. Use according to one of claims 1 to 21, wherein the composition comprises
(i) 10 to 60 wt.-% monomer/oligomer
(ii) 5 to 90 wt.-% filler.

23. Use according to one of claims 1 to 22, wherein the composition comprises 0.001 to 0.1 wt.% initiator.

24. Use of a kit that comprises the monomer/oligomer (a) and initiator-containing filler according to one of claims 1 to 23 in physically separated form for the preparation of a dental material.

25. Use according to one of claims 1 to 24 for the preparation of prosthetic plastics, false teeth, facing material or filling material.

## Revendications

1. Utilisation d'une composition contenant
(a) au moins un monomère et/ou un oligomère, qui est accessible à la polymérisation par métathèse avec ouverture de cycle,
(b) au moins une charge et
(c) au moins un initiateur pour la polymérisation par métathèse avec ouverture de cycle,
**caractérisée en ce que** l'initiateur est lié chimiquement ou physiquement à la charge au moyen d'un liant, pour fabriquer un matériau dentaire.

2. Utilisation selon la revendication 1, dans laquelle l'initiateur est lié chimiquement à la charge par un silane de formule (1) :
[(G-X)ᵣ-R¹-Y-R²]ₓ-SiR³_{y}R⁴_{z} Formule 1
dans laquelle les variables de la formule (1) ont les significations suivantes :
G est un radical organique linéaire ou ramifié pouvant être soumis à une métathèse, ayant m atomes de carbone, m étant un nombre entier de 1 à 40, et 0 à (m-2) hétéroatomes choisis parmi N, O, Si, P et S ; ou
un radical organique cycloaliphatique ou aromatique, cyclique ou polycyclique, pouvant être soumis à une métathèse, ayant m' atomes de carbone, m' étant un nombre entier de 3 à 63, et 0 à (m'-2) hétéroatomes choisis parmi N, 0, Si, P et S ;
X, Y représentent, indépendamment l'un de l'autre, -(C(=O)-O-, -C(=O)-N-, -O-C(=O)-O-, -O-C(=O)-NR⁵-, -CR⁵=N-, -O-, -S-, où R⁵ est un atome d'hydrogène, un alkyle en C₁-C₆, un benzyle ou est absent ;
R¹ est un radical organique aliphatique, cycloaliphatique ou aromatique, linéaire ou ramifié de valence (r+1) ou est absent ;
R² est un groupement alkylène en C₁-C₅ ou est absent;
R³ représente un halogène, un groupement hydroxy, alcoxy en C₁-C₅ ou alcoxyoxy en C₁-C₅ ou acyloxy en C₁-C₅;
R⁴ est un groupement alkyle en C₁-C₁₂, cycloalkyle en C₃-C₁₂ ou phényle ;
r est égal à 1, 2 ou 3 ;
x est égal à 1, 2 ou 3;
y est égal à 1, 2 ou 3;
z est égal à 0, 1 ou 2 ;
la somme de x+y+z étant de 4.

3. Utilisation selon la revendication 2, dans laquelle les variables de la formule (1) ont les significations suivantes:
G est un radical de formule (2) : 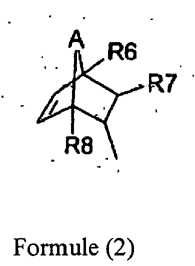
dans laquelle les variables sont définies comme suit :
A représente O, NH, S, un radical organique, saturé ou insaturé, avec t atomes de carbone, t étant un nombre entier de 1 à 12, qui peut contenir 0 à (t-1) hétéroatomes choisis parmi N, O, Si, P et S ;
R⁶, R⁷, R⁸ sont, respectivement, indépendamment l'un de l'autre, un radical aliphatique linéaire ou ramifié ayant q atomes de carbone, q étant un nombre entier de 1 à 15, un radical organique cyclique ou polycyclique ayant q' atomes de carbone, q' étant un nombre entier de 3 à 15, et 0 à (q-1) ou 0 à (q'-1) hétéroatomes choisis parmi N, O, Si, P et S;
X, Y sont absents ;
R¹ est un groupement alkyle en C₁-C₅ linéaire ou est absent ;
R² est absent ;
R³ est un atome de chlore, un groupement méthoxy, un groupement éthoxy ;
R⁴ est un groupement méthyle ;
r est égal à 1 ;
x est égal à 1 ;
y est égal à 1,2 ou 3 ;
z est égal à 0, 1 ou 2.

4. Utilisation selon la revendication 1, dans laquelle l'initiateur est lié physiquement à la charge par un silane de formule (3) :
(R⁹)ₐ(R¹⁰)_{b}(R¹¹)_{c}Si(R¹²)_{d} Formule (3)
où
R¹² est un halogène, un groupement hydroxy, alcoxy en C₁-C₅ ou acyloxy en C₁-C₅ ;
R⁹, R¹⁰, R¹¹ représentent, indépendamment l'un de l'autre, un radical organique linéaire ou ramifié, saturé ou insaturé, ayant s atomes de carbone, s étant un nombre entier de 2 à 20, et 0 à (s-1) hétéroatomes choisis parmi N, O, Si, P et S ; ou
un radical organique cyclique ou polycyclique, saturé, insaturé ou aromatique, ayant s' atomes de carbone, s' étant un nombre entier de 3 à 15, et 0 à (s'-1) hétéroatomes choisis parmi N, O, Si, P et S;
a est égal à 0, 1, 2 ou 3 ;
b est égal à 0, 1, 2 ou 3 ;
c est égal à 0, 1, 2 ou 3 ;
d est égal à 1, 2 ou 3;
la somme de a+b+c+d étant de 4.

5. Utilisation selon l'une quelconque des revendications 1 à 4, dans laquelle l'initiateur est un initiateur actif à température ambiante.

6. Utilisation selon la revendication 5, dans laquelle l'initiateur est un composé de formule (4) : 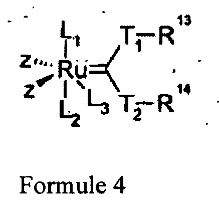
Z représente Cl, Br, F, I, un tosylate ou une des significations indiquées pour L₁, L₂, L₃ ;
L₁, L₂, L₃ représentent, indépendamment l'un de l'autre, P(R¹⁵)₃, où R¹⁵ est un groupement phényle, isopropyle, cyclohexyle ou 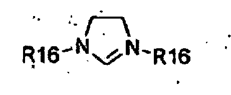 ou 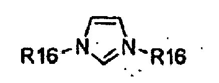 où R¹⁶ est un groupement mésityle (2,4,6-triméthylphényle; MES) ; ou
un groupement pyridine, qui est non substitué ou substitué en position 2 ou en positions 2 et 4 par Br, Cl, F, I, OCH₃, ou est absent ;
R¹³, R¹⁴ représentent, indépendamment l'un de l'autre, H ou un cycle en C₆-C₂₀ ou un système cyclique aromatique ou aliphatique, polycyclique ou condensé avec 0 à 5 hétéroatomes dans le cycle choisis parmi N, S, O et P, le cycle ou le système cyclique étant non substitué ou pouvant être substitué en plus par 0 à 5 substituants du type -Cl, -Br, -I, -F, -OR¹⁷, -CH=N-R¹⁷, -C(=O)R¹⁷, -C(-O)OR¹⁷, -OC(=O)R¹⁷, où R¹⁷ est un groupement alkyle en C₁-C₁₄ acyclique ou un groupement alkyle en C₃-C₁₄ cyclique, linéaire ou ramifié, ou un radical aromatique ayant 6 à 14 atomes de carbone, R¹⁷ pouvant contenir 0 à 5 hétéroatomes choisis parmi N, O, Si et S ;
T₁, T₂ représentent, indépendamment l'un de l'autre, -O-, -S- ou un groupement alkylène en C₁-C₄ saturé ou insaturé, qui peut contenir en plus 0 à 2 hétéroatomes du groupe constitué de N, O, S et Si ou sont absents.

7. Utilisation selon l'une quelconque des revendications 1 à 5, dans laquelle l'initiateur est un initiateur activable par voie thermique ou photochimique.

8. Utilisation selon la revendication 7, dans laquelle l'initiateur est : 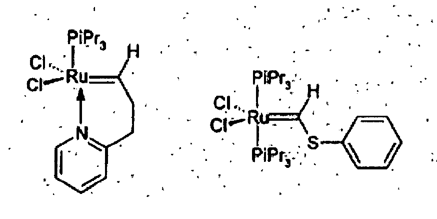 ou
un composé de formule (5) : 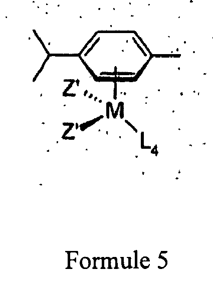 dans laquelle :
M représente Os ou Ru;
Z' représente Cl, Br, F, I, un tosylate ou une des significations indiquées pour L⁴ ;
L₄ est P(R¹⁸)₃, où R¹⁸ est un groupement phényle, isopropyle, 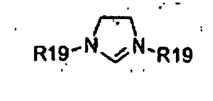 cyclohexyle ou
ou 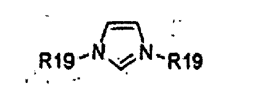 où R¹⁹ est un groupement mésityle (2,4,6-triméthylphényle).

9. Utilisation selon l'une quelconque des revendications 1 à 8, dans laquelle la charge contient 0,00001 à 0,1 mmole d'initiateur par gramme de charge.

10. Utilisation selon l'une quelconque des revendications 1 à 9, dans laquelle la composition contient un second composant de charge qui est modifié en surface par un silane de formule (1).

11. Utilisation selon l'une quelconque des revendications 1 à 10, dans laquelle la composition contient, comme composant (a), au moins un composé cyclique bi- ou multicyclique ayant au moins une double liaison endocyclique.

12. Utilisation selon la revendication 11, dans laquelle la composition contient un composé carbocyclique ou hétérocyclique de type hydrocarbures bicyclo[x',y',z'], x', y' et z' étant, respectivement, indépendamment l'un de l'autre, un nombre entier de 1 à 6.

13. Utilisation selon la revendication 12, dans laquelle la composition contient un dérivé de norbomène de formule (6) et/ou (7) : 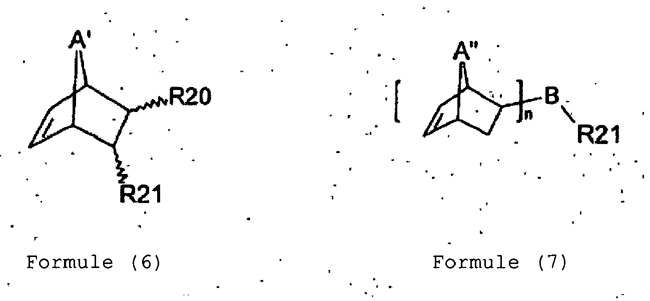 dans laquelle les variables ont les significations suivantes :
A', A" représentent O, S, NH, un radical organique, saturé ou insaturé, avec u atomes de carbone, u étant un nombre entier de 1 à 15, qui peut contenir 0 à (u-1) hétéroatomes choisis parmi N, O, Si, P et S;
B représente -CH₂- ou est absent ;
R²⁰, R²¹ représentent H ou un radical organique, saturé ou insaturé, ayant v atomes de carbone, v étant un nombre entier de 1 à 30, et 0 à (v-1) hétéroatomes choisis parmi N, O, Si, P, S et F ; ou
R²⁰ et R²¹ forment, conjointement avec les atomes auxquels ils sont liés, un système cyclique alicyclique condensé, saturé ou insaturé, de 4 à 12 atomes de carbone ou un système cyclique aromatique condensé de 6 à 12 atomes de carbone, qui peut être substitué à son tour par un alkyle en C₁-C₆ ou un benzyle ;
R²² représente un groupement alkylène en C₁-C₁₀, arylène en C₆-C₁₀, -O-C(=O)-phénylène-C(=O)-O-, (-C(=O)-)₄(C₆H₂), 2,4,6-trioxo-1,3,5-triazinyle, -O-C(=O)-(CH₂)ₘ-C(=O)-O-, -O-C(=O)-NH-(CH₂)ₘ-NH-C(=O)-O- substitué n fois, m étant un nombre entier de 1 à 10 ;
n représente un nombre entier de 1 à 4.

14. Utilisation selon l'une quelconque des revendications 1 à 13, dans laquelle l'initiateur est lié à une charge particulaire inorganique.

15. Utilisation selon la revendication 14, dans laquelle la charge particulaire est une charge ayant une taille particulaire primaire de 5 à 500 nm.

16. Utilisation selon la revendication 14 ou 15, dans laquelle la charge particulaire contient de l'acide silicique pyrogène, de l'acide silicique précipité, un oxyde mixte constitué de SiO₂, ZrO₂, TiO₂, TaO₂, La₂O₃, Yb₂O₃ et/ou CeO₂.

17. Utilisation selon la revendication 14, dans laquelle la charge particulaire présente une taille particulaire moyenne de 0,01 à 5 µm.

18. Utilisation selon la revendication 17, dans laquelle la charge particulaire contient des poudres de quartz, de vitrocéramique ou de verre.

19. Utilisation selon l'une quelconque des revendications 1 à 18, dans laquelle la composition contient du trifluorure d'ytterbium comme charge.

20. Utilisation selon l'une quelconque des revendications 1 à 19, dans laquelle la composition contient en outre :
(d) au moins un additif choisi parmi les substances suivantes : stabilisateur, absorbeur d'UV, colorant, pigment, agent lubrifiant.

21. Utilisation selon l'une quelconque des revendications 1 à 20, dans laquelle la composition est exempte de solvant.

22. Utilisation selon l'une quelconque des revendications 1 à 21, dans laquelle la composition contient :
(i) 10 à 60 % en poids de monomère/oligomère,
(ii) 5 à 90 % en poids de charge.

23. Utilisation selon l'une quelconque des revendications 1 à 22, dans laquelle la composition contient 0,001 à 0,1 % en poids d'initiateur.

24. Utilisation d'une trousse qui contient le monomère/oligomère (a) et une charge contenant un initiateur selon l'une quelconque des revendications 1 à 23 sous une forme spatialement séparée, pour fabriquer un matériau dentaire.

25. Utilisation selon l'une quelconque des revendications 1 à 24 pour fabriquer un matériau synthétique de prothèse, des dents artificielles, un matériau de revêtement ou un matériau d'obturation.
